(19) Europäisches Patentamt
European Patent Office
Office européen des brevets

(11) Veröffentlichungsnummer: **0 206 004 B1**

# (12) EUROPÄISCHE PATENTSCHRIFT

(45) Veröffentlichungstag der Patentschrift: **28.04.93**

(21) Anmeldenummer: **86107471.4**

(22) Anmeldetag: **02.06.86**

Die Akte enthält technische Angaben, die nach dem Eingang der Anmeldung eingereicht wurden und die nicht in dieser Patentschrift enthalten sind.

(51) Int. Cl.5: **C07C 249/04**, C07C 281/06, C07C 317/00, C07D 295/18, C07D 207/16, C07D 249/08

## (54) E-Isomere von N alpha-(2-Cyan-2-alkoximino-acetyl)-amino-säurederivaten und -peptiden.

(30) Priorität: **13.06.85 DE 3521131**
**25.01.86 DE 3602243**

(43) Veröffentlichungstag der Anmeldung:
**30.12.86 Patentblatt 86/52**

(45) Bekanntmachung des Hinweises auf die Patenterteilung:
**28.04.93 Patentblatt 93/17**

(84) Benannte Vertragsstaaten:
**AT BE CH DE FR GB IT LI NL SE**

(56) Entgegenhaltungen:
**EP-A- 201 999**
**EP-A- 0 000 023**
**DE-A- 2 312 956**

(73) Patentinhaber: **BAYER AG**
**W-5090 Leverkusen 1 Bayerwerk(DE)**

(72) Erfinder: **Lunkenheimer, Winfried, Dr.**
**Bismarckstrasse 29**
**W-5600 Wuppertal 1(DE)**
Erfinder: **Scheinpflug, Hans, Prof. Dr.**
**Am Thelenhof 15**
**W-5090 Leverkusen 1(DE)**
Erfinder: **Brandes, Wilhelm, Dr.**
**Eichendorffstrasse 3**
**W-5653 Leichlingen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Die vorliegende Erfindung betrifft neue E-Isomere von $N^{\alpha}$-(2-Cyan-2-alkoximinoacetyl)-aminosäurederivaten und -peptiden, mehrere Verfahren zu ihrer Herstellung und ihre Verwendung als Schädlingsbekämpfungsmittel, insbesondere als Fungizide.

Es ist bereits bekannt, daß bestimmte Verbindungen wie beispielsweise N-Trichlormethylthio-tetrahydrophthalimid, Zink-ethylen-1,2-bis-dithiocarbamat und N-Ethylaminocarbonyl-2-cyano-2-methoximino-acetamid gute fungizide Wirksamkeit besitzen (vgl. z.B. US-PS 2 553 770, US-PS 2 457 674 und DE-OS 2 312 956). Die Wirkung dieser Verbindungen ist jedoch, insbesondere bei niedrigen Aufwandmengen und -konzentrationen, nicht immer voll befriedigend.

Es wurden neue E-Isomere von $N^{\alpha}$-(2-Cyan-2-alkoximino-acetyl)-aminosäurederivaten und -peptiden der allgemeinen Formel (I)

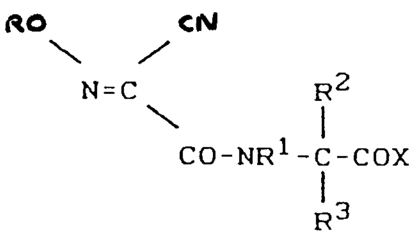

(I)

in welcher

R für geradkettiges oder verzweigtes Alkyl mit 1 bis 8 Kohlenstoffatomen, Alkenyl oder Alkinyl mit jeweils 2 bis 4 Kohlenstoffatomen, für Cyanalkyl mit 1 bis 4 Kohlenstoffatomen, für gegebenenfalls im Phenylteil einfach bis dreifach, gleich oder verschieden substituiertes Phenylalkyl mit 1 bis 4 Kohlenstoffatomen im Alkylteil steht, wobei als Substituenten vorzugsweise genannt seien: Halogen, Cyano, Nitro, Hydroxy, Alkyl, Alkoxy, Alkylsulfinyl und Alkylsulfonyl, Alkylthio mit jeweils 1 bis 4 Kohlenstoffatomen und gegebenenfalls einfach bis dreifach, gleich oder verschieden durch Halogen substituiertes Phenyl; oder für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien;

$R^1$ für Wasserstoff, geradkettiges oder verzweigtes Alkyl mit 1 bis 4 Kohlenstoffatomen, für jeweils gegebenenfalls einfach bis dreifach, gleich oder verschieden substituiertes Phenyl oder Benzyl steht, wobei als Substituenten vorzugsweise die bei R bereits genannten Phenylsubstituenten in Frage kommen, oder für Alkoxycarbonylamino mit 1 bis 4 Kohlenstoffatomen im Alkylteil steht;

$R^2$ für Wasserstoff oder geradkettiges oder verzweigtes Alkyl mit 1 bis 4 Kohlenstoffatomen steht;

$R^3$ für Wasserstoff, geradkettiges oder verzweigtes Alkyl mit 1 bis 6 Kohlenstoffatomen, für geradkettiges oder verzweigtes Hydroxyalkyl mit 1 bis 4 Kohlenstoffatomen, für Alkoxycarbonylalkyl mit jeweils 1 bis 4 Kohlenstoffatomen im Alkoxyteil und im Alkylteil, für Hydroxycarbonylalkyl und Aminocarbonylalkyl mit jeweils 1 bis 4 Kohlenstoffatomen im Alkylteil, für Cyanalkyl mit 1 bis 4 Kohlenstoffatomen, für Alkenyl oder Alkinyl mit jeweils 2 bis 4 Kohlenstoffatomen, für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien, ferner für gegebenenfalls im Phenylteil einfach bis dreifach, gleich oder verschieden substituiertes Phenyl oder Phenylalkyl mit 1 bis 4 Kohlenstoffatomen im Alkylteil steht, wobei als Substituenten jeweils vorzugsweise die bei R bereits genannten Phenylsubstituenten in Frage kommen,
oder für die Gruppierung

$R^4$-$SO_n$-Z-

steht, wobei

$R^4$ für Wasserstoff, geradkettiges oder verzweigtes Alkyl mit 1 bis 4 Kohlenstoffatomen, für gegebenenfalls im Phenylteil einfach bis dreifach, gleich oder verschieden substituiertes Phenylalkyl mit 1 bis 4 Kohlenstoffatomen im Alkylteil steht, wobei als Substituenten die bei R bereits genannten Phenylsubstituenten in Frage kommen,

n für die Zahlen 0, 1 oder 2 steht und

Z für eine geradkettige oder verzweigte Alkylenkette mit 1 bis 4 Kohlenstoffatomen steht;

$R^2$ und $R^3$ gemeinsam mit dem Kohlenstoffatom, an das sie gebunden sind, für Cycloalkyliden mit 3 bis 6 Kohlenstoffatomen stehen;

X für die Gruppierungen $-OR^I$ oder $-NR^{II}R^{III}$ steht, wobei

$R^I$ für Wasserstoff steht;

$R^{II}$ für Wasserstoff oder geradkettiges oder verzweigtes Alkyl mit 1 bis 4 Kohlenstoffatomen steht;

$R^{III}$ geradkettiges oder verzweigtes Alkyl mit 1 bis 4 Kohlenstoffatomen, für Alkenyl mit 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit bis zu 4 Kohlenstoffatomen in den einzelnen Alkylteilen, für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil,für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei R bereits genannten Phenylsubstituenten in Frage kommen, für gegebenenfalls gleich oder verschieden substituiertes Cycloalkyl mit 3 bis 7 Kohlenstoffatomen, wobei als Substituenten vorzugsweise Halogen und Alkyl mit 1 bis 4 Kohlenstoffatomen in Frage kommen, für Alkylcarbonylamino oder Alkoxycarbonylamino mit jeweils 1 bis 4 Kohlenstoffatomen im Alkylteil beziehungsweise im Alkoxyteil, außerdem für Aminocarbonylamino, Alkylaminocarbonylamino und Dialkylaminocarbonylamino mit jeweils 1 bis 4 Kohlenstoffatomen in den einzelnen Alkylteilen sowie für Formylamino oder für die Gruppierung

$$-N=CH-R^{IV}$$

steht, wobei

$R^{IV}$ für geradkettiges oder verzweigtes Alkyl mit 1 bis 4 Kohlenstoffatomen oder gegebenenfalls einfach bis dreifach, gleich oder verschieden substituiertes Phenyl steht, wobei als Substituenten die bei R bereits genannten Phenylsubstituenten in Frage kommen,

oder

$R^{II}$ und $R^{III}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl sowie Alkylaminocarbonyl und Dialkylaminocarbonyl mit jeweils 1 bis 4 Kohlenstoffatomen in den einzelnen Alkylteilen substituiert sein kann,

sowie deren physiologisch verträglichen gegebenenfalls substituierten Ammonium-, Alkali- und Erdalkali-Salze sowie Metallsalz-Komplexe,
ausgenommen Verbindungen,
in denen $R^{III}$ für Alkyl steht,
wenn $R^1$ und $R^{II}$ für Wasserstoff stehen.

Die Verbindungen der Formel (I) besitzen für den Fall, daß $R^2$ und $R^3$ unterschiedliche Bedeutung haben, ein asymmetrisches Kohlenstoffatom; sie können somit auch als optische Isomere (D- und L-Konfiguration) vorliegen, die in unterschiedlichen Mengenverhältnissen anfallen können. Vorwiegend fallen sie als Racemate an.

Weiterhin wurde gefunden, daß man die E-Isomeren von $N^{\alpha}$-(2-Cyan-2-alkoximinoacetyl)-aminosäurederivaten und -peptiden der Formel (I)

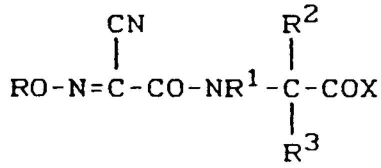 (I)

in welcher
R, $R^1$, $R^2$, $R^3$ und X die oben angegebene Bedeutung haben,
sowie deren physiologisch verträglichen gegebenenfalls substituierten Ammonium-, Alkali- und Erdalkali-Salze sowie Metallsalz -Komplexe erhält, wenn man

(a) Aminosäure-Derivate der Formel (II)

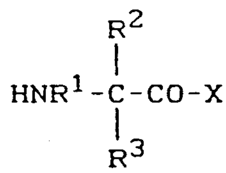 (II)

in welcher $R^1$, $R^2$, $R^3$ und X die oben angegebenen Bedeutungen haben, mit den carboxy-aktivierten Derivaten der E-Isomeren der Carbonsäuren der Formel (III)

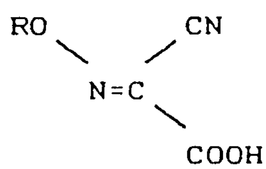 (III)

in welcher R die oben angegebene Bedeutung hat, gegebenenfalls in Gegenwart eines Katalysators, gegebenenfalls in Gegenwart eines Säurebindemittels und gegebenenfalls in Gegenwart eines Verdünnungsmittels umsetzt,
oder

(b) carboxy-aktivierte Derivate der E-Isomeren von Carbonsäuren der Formel (III)

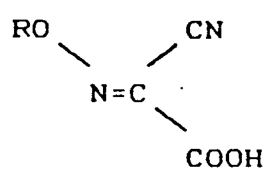 (III)

in welcher R die oben angegebene Bedeutung hat, zunächst mit Aminen der Formel (IV)

$H_2NR^1$ (IV)

in welcher $R^1$ die oben angegebene Bedeutung hat, anschließend mit Carbonyl-Derivaten der Formel (V)

$R^2$-CO-$R^3$ (V)

in welcher $R^2$ und $R^3$ die oben angegebenen Bedeutungen haben,
und schließlich mit Isonitrilen der Formel (VI)

CN-$R^5$ (VI)

in welcher
$R^5$ für Alkyl, Alkenyl, Alkinyl, Cyanoalkyl, Alkoxycarbonylalkyl oder für gegebenenfalls substituiertes Cycloalkyl, Phenyl oder Phenylalkyl steht,
gegebenenfalls in Gegenwart eines Verdünnungsmittels umsetzt, oder
(c) carboxy-aktivierte Derivate der E-Isomeren von Aminosäuren der Formel (Ia)

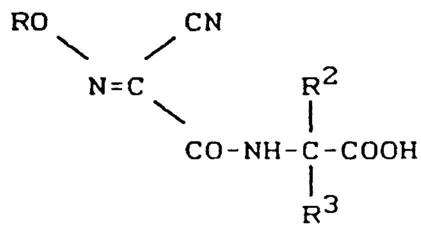

(Ia)

in welcher
R, $R^2$ und $R^3$ die oben angegebenen Bedeutungen haben,
mit Aminen der Formel (VII)

H-$NR^{II}R^{III}$ (VII)

in welcher
$R^{II}$ und $R^{III}$ die oben angegebenen Bedeutungen haben,
gegebenenfalls in Gegenwart eines Katalysators und gegebenenfalls in Gegenwart eines Säurebindemittels und gegebenenfalls in Gegenwart eines Verdünnungsmittels umsetzt,
oder
(d) E-Isomere von Aminosäurederivaten der Formel (Ib)

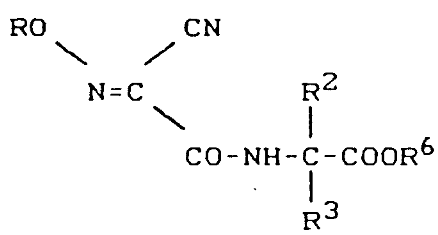

(Ib)

in welcher
R, $R^2$ und $R^3$ die oben angegebenen Bedeutungen haben, und
$R^6$ für Alkyl mit 1 bis 4 Kohlenstoffatomen steht,
mit Aminen der Formel (VII)

H-$NR^{II}R^{III}$ (VII)

in welcher
$R^{II}$ und $R^{III}$ die oben angegebenen Bedeutungen haben,
gegebenenfalls in Gegenwart eines Verdünnungsmittels umsetzt.

Die so zu erhaltenen Verbindungen der Formel (I), in denen X für eine Hydroxy-Gruppe steht, können mit Aminen und Alkali- bzw. Erdalkalihydroxiden, -carbonaten oder -hydrogencarbonaten sowie Ammoniumhydroxid Salze bilden -, außerdem können sie mit Schwermetallsalzen Metall-Komplexe bilden.

Schließlich wurde gefunden, daß die neuen E-Isomeren von $N^{\alpha}$-(2-Cyan-2-alkoximinoacetyl)-aminosäurederivaten und -peptiden der Formel (I) sowie deren gegebenenfalls substituierten Ammonium-, Alkali- und Erdalkali-Salze sowie Metallsalz-Komplexe insbesondere starke fungizide Eigenschaften aufweisen Dabei zeigen überraschenderweise die erfindungsgemäßen Verbindungen eine höhere Wirkung als die aus dem Stand der Technik bekannten Verbindungen, wie das N-Trichlormethylthio-tetrahydrophthalimid, das Zinkethylen-1,2-bis-dithiocarbamat und das N-Ethylaminocarbonyl-2-cyano-2-methoximinoacetamid, welche

konstitutionell beziehungsweise wirkungsmäßig naheliegende Verbindungen sind. Die erfindungsgemäßen Stoffe stellen somit eine Bereicherung der Technik dar.

Die erfindungsgemäßen E-Isomeren von $N^{\alpha}$-(2-Cyan-2-alkoximinoacetyl)-aminosäurederivaten und -peptiden sind durch die Formel (I) allgemein definiert. Bevorzugt sind Verbindungen der Formel (I), in denen

R für geradkettiges oder verzweigtes Alkyl mit 1 bis 4 Kohlenstoffatomen, Allyl, Propargyl, Cyanmethyl, Cyanethyl, für gegebenenfalls im Phenylteil einfach oder zweifach, gleich oder verschieden substituiertes Phenylalkyl mit 1 oder 2 Kohlenstoffatomen im Alkylteil steht, wobei als Substituenten genannt seien: Fluor, Chlor, Cyano, Nitro, Hydroxy, Methyl, Methoxy, Methylthio, Methylsulfinyl, Methylsulfonyl und gegebenenfalls einfach oder zweifach, gleich oder verschieden durch Fluor oder Chlor substituiertes Phenyl; ferner für jeweils gegebenenfalls einfach oder zweifach, gleich oder verschieden durch Fluor, Chlor oder Methyl substituiertes Cyclopropyl, Cyclobutyl, Cyclopentyl, Cylohexyl oder Cycloheptyl steht;

$R^1$ für Wasserstoff, Methyl, Ethyl, für jeweils gegebenenfalls einfach oder zweifach, gleich oder verschieden substituiertes Phenyl oder Benzyl steht, wobei als Substituenten insbesondere die bei R bereits genannten Phenylsubstituenten in Frage kommen; ferner für Methoxycarbonylamino oder Ethoxycarbonylamino steht;

$R^2$ für Wasserstoff, Methyl oder Ethyl steht;

$R^3$ für Wasserstoff, geradkettiges oder verzweigtes Alkyl mit 1 bis 4 Kohlenstoffatomen, Hydroxymethyl, 1-Hydroxyethyl, für Methoxycarbonylmethyl, Methoxycarbonylethyl, Ethoxycarbonylmethyl, Ethoxycarbonylethyl, Hydroxycarbonylmethyl, Hydroxycarbonylethyl, Aminocarbonylmethyl, Aminocarbonylethyl, für Cyanmethyl, Cyanethyl, für Allyl, für Propargyl, für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht; ferner für gegebenenfalls im Phenylteil einfach oder zweifach, gleich oder verschieden substituiertes Phenylalkyl mit 1 oder 2 Kohlenstoffatomen im Alkylteil oder einfach oder zweifach, gleich oder verschieden substituiertes Phenyl steht, wobei als Substituenten die bei R bereits genannten Phenylsubstituenten in Frage kommen, ferner für die Gruppierung

$R^4$-$SO_n$-Z-

steht, wobei

$R^4$ für Wasserstoff, Methyl, Ethyl oder für gegebenenfalls im Phenylteil einfach oder zweifach, gleich oder verschieden substituiertes Phenylalkyl mit 1 oder 2 Kohlenstoffatomen im Alkylteil steht, wobei als Substituenten insbesondere die bei R bereits genannten Phenylsubstituenten in Frage kommen,

n für die Zahlen 0, 1 oder 2 steht und

Z für eine geradkettige oder verzweigte Alkylenkette mit 1 oder 2 Kohlenstoffatomen steht;

$R^2$ und $R^3$ gemeinsam mit dem Kohlenstoffatom, an das sie gebunden sind, für Cyclopropyliden stehen, und

X für die Gruppierungen $-OR^I$ oder $-NR^{II}R^{III}$ steht, wobei

$R^I$ für Wasserstoff;

$R^{II}$ für Wasserstoff, Methyl oder Ethyl steht;

$R^{III}$ für Methyl, Ethyl, n-Propyl, i-Propyl, n-Butyl, i-Butyl, sec.-Butyl, tert.-Butyl, Allyl, für Halogenalkyl mit 1 oder 2 Kohlenstoffatomen und 1 bis 3 gleichen oder verschiedenen Halogenatomen, wie Fluor- und Chloratomen, für Alkoxyalkyl mit jeweils 1 oder 2 Kohlenstoffatomen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanoalkyl; ferner für gegebenenfalls im Phenylteil einfach oder zweifach, gleich oder verschieden substituiertes Phenylalkyl mit 1 oder 2 Kohlenstoffatomen im Alkylteil, für einfach oder zweifach, gleich oder verschieden substituiertes Phenyl steht, wobei als Substituenten jeweils die bei R bereits genannten Phenylsubstituenten in Frage kommen; für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl, für Alkylcarbonylamino oder Alkoxycarbonylamino mit jeweils 1 oder 2 Kohlenstoffatomen im Alkylteil beziehungsweise im Alkoxyteil, für Aminocarbonylamino, Methylaminocarbonylamino, Ethylaminocarbonylamino, Dimethylaminocarbonylamino, Diethylaminocarbonylamino, Methylethylaminocarbonylamino sowie für Formylamino oder für die Gruppierung

$-N=CH-R^{IV}$

steht, wobei

$R^{IV}$ für Methyl, Ethyl, für gegebenenfalls ein- oder zweifach, gleich oder verschieden substituiertes Phenyl steht, wobei als Substituenten die bei R bereits vorzugsweise genannten Phenylsubstituenten in Frage kommen; oder

$R^{II}$ und $R^{III}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann und gegebenenfalls durch Cyano, Fluor, Chlor, Brom, Iod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl sowie Methylethylaminocarbonyl substituiert sein kann,

ausgenommen Verbindungen, in denen $R^{III}$ für Alkyl steht, wenn $R^1$ und $R^{II}$ für Wasserstoff stehen.

Bevorzugte erfindungsgemäße Verbindungen sind auch die Alkali-, Erdalkali- und gegebenenfalls substituierten Ammoniumsalze der E-Isomeren derjenigen $N^{\alpha}$-(2-Cyan-2-alkoximinoacetyl)-aminosäure- und -peptid-Derivate der Formel (I), in denen X für eine Hydroxygruppe steht und R, $R^1$, $R^2$ und $R^3$ die Bedeutungen haben, die bereits für diese Substituenten genannt wurden.

Für diese Salzbildung können Alkali- und Erdalkalihydroxide, -carbonate oder -hydrogencarbonate oder Amine und Ammoniumhydroxid eingesetzt werden. Hierzu gehören vorzugsweise Natriumhydroxid, Natriumcarbonat, Natriumhydrogencarbonat, Kaliumhydroxid, Kaliumcarbonat, Calciumhydroxid, Bariumhydroxid; Ammoniak; primäre Amine, wie Methylamin und Isopropylamin; sekundäre Amine, wie Dimethylamin und Dicyclohexylamin; tertiäre Amine, wie Triethylamin, Pyridin und N,N-Dimethylbenzylamin sowie Ammoniumhydroxide, wie Benzyltrimethyl-ammoniumhydroxid.

Außerdem bevorzugte erfindungsgemäße Verbindungen sind Additionsprodukte, aus Salzen von Metallen der II. bis IV. Haupt- und der I. und II. sowie IV. bis VIII. Nebengruppe und denjenigen substituierten $N^{\alpha}$-(2-Cyan-alkoxaminoacetyl)-aminosäure- und - peptid-Derivaten der Formel (I), in denen die Substituenten R, $R^1$, $R^2$, $R^3$ und X die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise Glycinethylester und 2-Cyan-2-methoximino-acetylchlorid als Ausgangsstoffe, so kann der Ablauf des Verfahrens (a) durch das folgende Formelschema wiedergegeben werden:

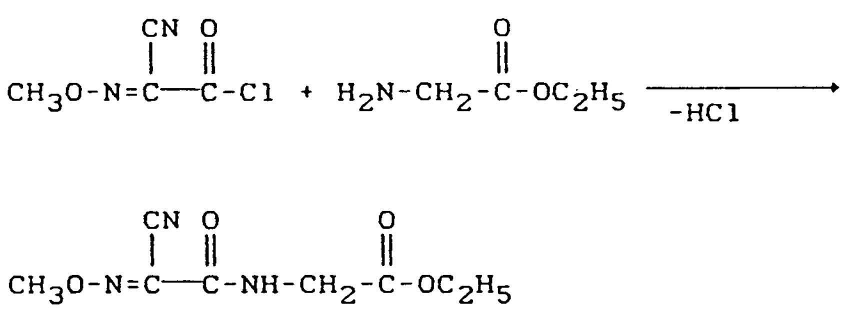


Verwendet man beispielsweise 2-Cyan-2-methoximinoessigsäure, Benzylamin, Isobutyraldehyd und Isocyanessigsäureethylester als Ausgangsprodukte, so kann der Ablauf des Verfahrens (b) durch das folgende Formelschema wiedergegeben werden:

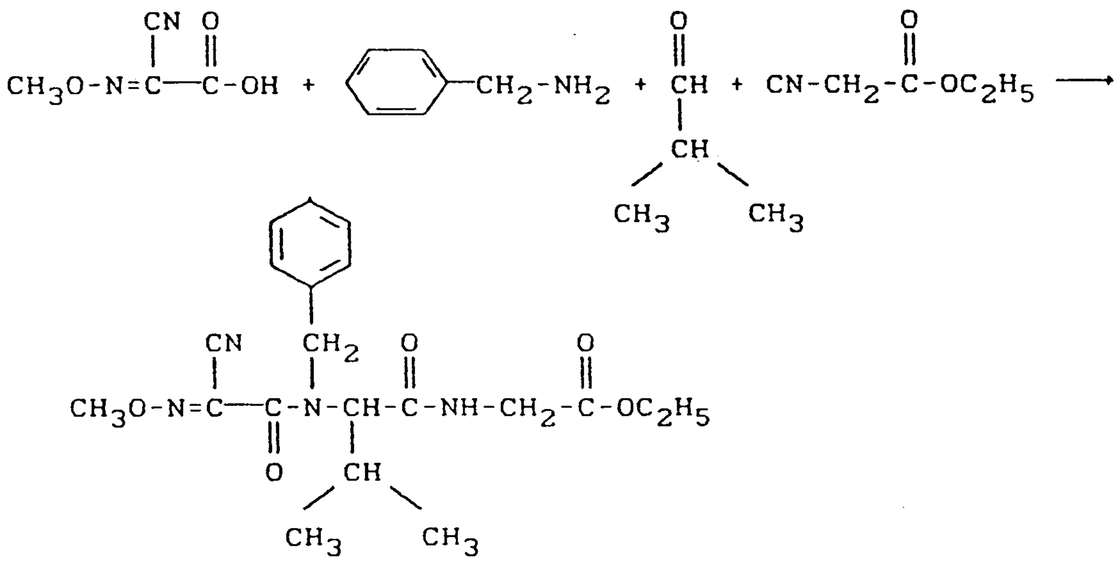

Verwendet man beispielsweise N-(2-Cyan-2-methoximinoacetyl)-glycinethylester und Methylamin als Ausgangsprodukte, so kann der Ablauf des Verfahrens (c) durch das folgende Formelschema wiedergegeben werden:

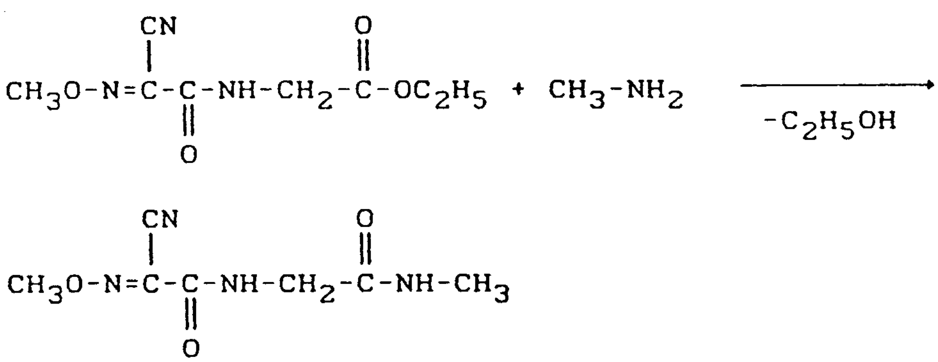


Die für die Durchführung des erfindungsgemäßen Verfahrens (a) als Ausgangsstoffe zu verwendenden Aminosäure-Derivate sind durch die Formel (II) allgemein definiert. In dieser Formel haben $R^1$, $R^2$, $R^3$ und X die Bedeutungen, die bereits im Zusammenhang mit der Beschreibung der erfindungsmäßen Stoffe der Formel (I) für diese Substituenten genannt wurden.

Die Aminosäure-Derivate der Formel (II) sind teilweise bekannt (vgl. z.B. Houben-Weyl, Methoden der organischen Chemie, Band XV, Teil 1 und 2, Georg Thieme Verlag, Stuttgart 1974; bzw. R.C. Sheppard, A Specialist Periodical Report, Amino-acids, Peptids and Proteins, The Royal Society of Chemistry, Burlington House, London 1978, bzw. I.P. Greenstein and M. Winitz, Chemistry of Amino Acids, I. Wiley Sons Inc., New York, London 1961; bzw. E. Schröder and K. Lübke, The Peptides Vol. I, Academic Press, New York, London 1965) oder können nach den dort angegebenen Verfahren erhalten werden.

Die außerdem für die Durchführung des erfindungsgemäßen Verfahrens (a) und ebenfalls des Verfahrens (b) als Ausgangsstoffe zu verwendenden carboxy-aktivierten Derivate der E-Isomeren der Carbonsäuren sind durch die Formel (III) allgemein definiert. In dieser Formel hat R die oben genannte Bedeutung.

Die carboxy-aktivierten Derivate der E-Isomeren der Carbonsäuren der Formel (III) sind, ausgenommen das E-Isomere der 2-Cyan-2-methoximino-essigsäure (vgl. I.J. Belasco und F.J. Baude, Pestic. Sci., 12, 27, 1981) nicht bekannt. Man erhält die carboxy-aktivierten Derivate der Carbonsäuren der Formel (III), wenn man nach allgemein bekannten Verfahren die Carbonsäureester der Formel (VIII)

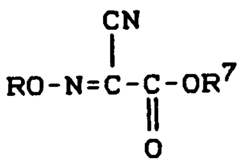

(VIII)

in welcher

R die oben angegebene Bedeutung hat und

$R^7$ für Alkyl, vorzugsweise Methyl oder Ethyl, steht,

zunächst mit einem Alkalihydroxid, wie beispielsweise Natriumhydroxid, und anschließend mit einer Säure, wie beispielsweise Salzsäure oder Schwefelsäure, oder mit einen sauren Ionenaustauscher in Gegenwart eines Verdünnungsmittels, wie beispielsweise Wasser, Alkohole, Ether oder Gemische von Alkoholen oder Ethern mit Wasser, bei Temperaturen zwischen 0 und 80° C, vorzugsweise zwischen 20 und 40° C, umsetzt.

Vorzugsweise erfolgt die Verseifung mit Kaliumhydroxid in Gegenwart eines Wasser/Ethanol-Gemisches, wobei das Reaktionsgemisch bei 20° C im Vakuum eingedampft wird und das entstehende Kaliumsalz mit z.B. Oxalylchlorid zum entsprechenden Säurechlorid umgesetzt wird (vgl. auch die Herstellungsbeispiele).

Die Carbonsäureester der Formel (VIII) sind teilweise bekannt (vgl. I.J. Belasco und F.J. Baude, Pestic, Sci., 12, 27, 1981) oder können nach dem dort beschriebenen Verfahren erhalten werden, in dem man die E-Isomeren der Carbonsäureester der Formel (IX)

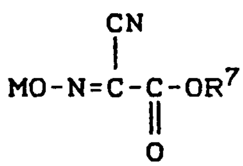

(IX)

in welcher

$R^7$ die oben genannte Bedeutung hat und

M für Wasserstoff, Natrium oder Kalium, vorzugsweise für Natrium steht,

mit einer Verbindung der Formel (X)

R-W (X)

in welcher

R die oben angegebene Bedeutung hat und

W für Halogen, einen Sulfonyldioxy- oder Sulfonyloxy-Rest, wie vorzugsweise Chlor, Brom, Jod, $-OSO_2OCH_3$, $-OSO_2OC_2H_5$, $-OSO_2CH_3$ oder

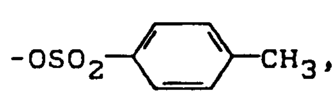

steht,

gegebenenfalls in Gegenwart einer Base, wie beispielsweise Kaliumcarbonat, Triethylamin oder Diazabicycloundecan (DBU), und in Gegenwart eines Lösungsmittels, wie beispielsweise Aceton, Dimethylformamid, Dimethylsulfoxid oder Acetonitril, bei Temperaturen zwischen 0 und 120° C umsetzt.

Die E-Isomeren der Carbonsäureester der Formel (IX) sind bekannt (vgl. z.B. G. Kinast, Liebigs Ann. Chem., 1981, 1561; M. Conrad und A. Schluze, Ber. dtsch. chem. Ges., 42, 735, 1909) und können nach den dort beschriebenen Verfahren erhalten werden.

Die Verbindungen der Formel (X) sind allgemein bekannte Alkylierungsmittel.

Als carboxy-aktivierte Derivate der Carbonsäuren der Formel (III) kommen alle Carboxy-aktivierten Derivate infrage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie mit Kondensationsmitteln, wie z.B. Dicyclohexylcarbodiimid oder Carbonyldiimidazol, in situ erzeugte aktivierte Formen der Carbonsäuren.

Vorzugsweise werden die den Carbonsäuren der Formel (III) entsprechenden Säurechloride eingesetzt. Sie können hergestellt werden, indem man die Carbonsäuren der Formel (III) oder deren Salze mit einem Halogenierungsmittel, wie beispielsweise Phosphorpentachlorid, Thionylchlorid oder Oxalylchlorid, in allgemein bekannter Art und Weise umsetzt. Bevorzugt ist der Einsatz von Oxalylchlorid zusammen mit dem Natrium- oder Kaliumsalz der Carbonsäure der Formel (III), da unter diesen Bedingungen die Isomerisierung minimal ist.

Die Acylierung mit den carboxy-aktivierten Derivaten kann in wäßrigem oder nichtwäßrigem Medium durchgeführt werden; geeignete Medien sind dabei Ketone, wie z.B. Aceton; Ester, wie z.B. Ethylacetat; Amide, wie z.B. Diemthylformamid; Nitrile, wie z.B. Acetonitril; Chlorkohlenwasserstoffe, wie z.B. Methylenchlorid, Kohlenwasserstoffe, wie z.B. Toluol; oder Ether, wie z.B. Tetrahydrofuran bzw. deren Mischungen.

Die Acylierung mit einem Säurehalogenid kann in Gegenwart eines Säurebindemittels, wie vorzugsweise eines tertiären Amins, wie z.B. Triethylamin, Pyridin, oder einer anorganischen Base, wie z.B. Natriumcarbonat oder Calciumcarbonat, durchgeführt werden.

Die für die Durchführung des erfindungsgemäßen Verfahrens (b) als Ausgangsstoffe zu verwendenden Isonitrile sind durch die Formel (VI) allgemein definiert. In dieser Formel steht $R^5$ vorzugsweise für geradkettiges oder verzweigtes Alkyl mit 1 bis 4 Kohlenstoffatomen, für Alkenyl oder Alkinyl mit jeweils 2 bis 4 Kohlenstoffatomen für Cyanoalkyl mit 1 bis 4 Kohlenstoffatomen, im Alkylteil, für Alkoxycarbonylalkyl mit jeweils 1 bis 4 Kohlenstoffatomen im Alkoxyteil und im Alkylteil, ferner für gegebenenfalls einfach bis dreifach, gleich oder verschieden substituiertes Cycloalkyl mit 3 bis 7 Kohlenstoffatomen, wobei als Substituenten vorzugsweise Halogen und Alkyl mit 1 bis 4 Kohlenstoffatomen genannt seien, oder für gegebenenfalls im Phenylteil einfach bis dreifach, gleich oder verschieden substituiertes Phenylalkyl mit 1 bis 4 Kohlenstoffatomen im Alkylteil oder für einfach bis dreifach, gleich oder verschieden substituiertes Phenyl, wobei als Substituenten jeweils vorzugsweise die bei R bereits vorzugsweise genannten Phenylalkylsubstituenten in Frage kommen.

Die Isonitrile der Formel (VI) sind teilweise bekannt (vgl. z.B. I.Ugi, Isonitrile Chemistry, Academic Press, New York, 1971) bzw. können sie in analoger Weise nach allgemein bekannten Verfahren hergestellt werden, indem man die entsprechenden formylierten primären Amine beispielsweise mit Phosgen oder Phosphoroxichlorid in Gegenwart eines tertiären Amins, wie z.B. Pyridin oder Triethylamin, unter Wasserabspaltung umsetzt.

Die außerdem für die Durchführung des erfindungsgemäßen Verfahrens (b) als Ausgangsstoffe zu verwendenden Amine und Carbonyl-Derivate sind durch die Formel (IV) beziehungsweise (V) allgemein definiert. In diesen Formeln haben $R^1$ bzw. $R^2$ und $R^3$ die oben genannten Bedeutungen.

Die Amine der Formel (IV) und die Carbonyl-Derivate der Formel (V) sind allgemein bekannte Verbindungen der organischen Chemie.

Die für die Durchführung des erfindungsgemäßen Verfahrens (c) als Ausgangsstoffe zu verwendenden Aminosäure-Derivate, die Teil der Erfindung sind, sind durch die Formel (Ia) allgemein definiert. In dieser Formel haben R, $R^2$ und $R^3$ vorzugsweise die Bedeutung, die bereits im Zusammenhang mit der Beschreibung der erfindungsgemäßen Stoffe der Formel (I) für diese Substituenten genannt wurden.

Als Acylierungsmittel entsprechend den Aminosäure-Derivaten der Formel (Ia) kommen alle carboxy-aktivierten Derivate in Frage, die im Zusammenhang mit den Acylierungsmitteln entsprechend den Carbonsäuren-Derivaten der Formel (III) bereits genannt wurden, vorzugsweise Azide, O-Alkylkohlensäureanhydride sowie mit Dicyclohexylcarbodiimid in-situ erzeugte aktivierte Derivate.

Die Aminosäure-Derivate der Formel (Ia) sind erfindungsgemäße Verbindungen und können gemäß dem erfindungsgemäßen Verfahren (a) hergestellt werden.

Die außerdem für die Durchführung des erfindungsgemäßen Verfahrens (c) als Ausgangsstoffe zu verwendenden Amine sind durch die Formel (VII) allgemein definiert. In dieser Formel haben $R^{II}$ und $R^{III}$ die obengenannten Bedeutungen.

Die Amine der Formel (VII) sind allgemein bekannte Verbindungen der organischen Chemie.

Die für die Durchführung des erfindungsgemäßen Verfahrens (d) als Ausgangsstoffe zu verwendenden Aminosäurederivate sind durch die Formel (Ib) allgemein definiert. In dieser Formel haben R, $R^2$ und $R^3$ vorzugsweise die Bedeutungen, die bereits im Zusammenhang mit der Beschreibung der erfindungsgemäßen Stoffe der Formel (I) für diese Substituenten genannt wurden. $R^6$ steht vorzugsweise für Methyl oder Ethyl.

Die Aminosäurederivate der Formel (Ib) sind erfindungsgemäße Verbindungen.

Als Verdünnungsmittel kommen für das erfindungsgemäße Verfahren (a) Wasser und inerte, organische Lösungsmittel in Frage. Hierzu gehören Ketone, wie Aceton oder Ethylmethylketon; Ester wie Ethyl- oder Methylacetat; Amide wie Dimethylformamid; Nitrile wie Acetonitril; Chlorkohlenwasserstoffe, wie Methylenchlorid oder Tetrachlorkohlenstoff; Kohlenwasserstoffe, wie Toluol; oder Ether, wie Tetrahydrofuran; bzw. deren Mischungen.

Als Säurebindemittel kommen für das erfindungsgemäße Verfahren (a) übliche anorganische und organische Säurebinder in Frage, Hierzu gehören vorzugsweise tertiäre Amine, wie Triethylamin, Pyridin oder N-Methylmorpholin; sowie anorganische Basen, wie Natriumcarbonat oder Calciumcarbonat.

Das erfindungsgemäße Verfahren (a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung des Verfahrens (a) in einem größeren Bereich variiert werden. Im allgemeinen arbeitet man zwischen -60 bis +120° C, vorzugsweise bei -20 bis +40° C.

Bei der Durchführung des erfindungsgemäßen Verfahrens (a) arbeitet man vorzugsweise in äquimolaren Mengen.

Dabei werden die Aminosäurederivate der Formel (II) als reine optische Isomere (D bzw. L-Form) oder als Racemate eingesetzt.

Als Verdünnungsmittel kommen für das erfindungsgemäße Verfahren (b) alle üblichen inerten organischen Lösungsmittel in Frage. Hierzu gehören vorzugsweise Alkohole, wie Methanol oder Ethanol; Ether, wie Diethylether; chlorierte Kohlenwasserstoffe, wie Chloroform oder Tetrachlorkohlenstoff; sowie Ketone, wie Aceton.

Die Temperaturen können bei der Durchführung des erfindungsgemäßen Verfahrens (b) in einem größeren Bereich variiert werden. Im allgemeinen arbeitet man zwischen -20 bis +40° C, vorzugsweise bei 0 bis 20° C.

Bei der Durchführung des erfindungsgemäßen Verfahrens (b) arbeitet man vorzugsweise in äquimolaren Mengen.

Die für das erfindungsgemäße Verfahren (c) in Frage kommenden Verdünnungsmittel, Katalysatoren und Säurebindemittel entsprechen denen des Verfahrens (a).

Die Temperaturen können bei der Durchführung des erfindungsgemäßen Verfahrens (c) in einem größeren Bereich variiert werden. Im allgemeinen arbeitet man zwischen -60 bis +120° C, vorzugsweise bei -20 bis +40° C.

Bei der Durchführung des erfindungsgemäßen Verfahrens (c) arbeitet man vorzugsweise in äquimolaren Mengen.

Als Verdünnungsmittel kommen für das erfindungsgemäße Verfahren (d) vorzugsweise Alkohole in Frage, wie beispielsweise Methanol, Ethanol oder Isopropanol.

Die Temperaturen können bei der Durchführung des erfindungsgemäßen Verfahrens (d) in einem größeren Bereich variiert werden. Im allgemeinen arbeitet man zwischen -20 und +60° C, vorzugsweise bei 0 bis 20° C.

Bei der Durchführung des erfindungsgemäßen Verfahrens (d) setzt man vorzugsweise auf 1 Mol der Verbindungen der Formel (Ib) 1 bis 10 Mol Amin der Formel (VII) ein.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet.

Zum Beispiel werden fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt.

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;

Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Schädlingsbekämpfungsmittel können die erfindungsgemäßen Wirkstoffe mit besonders gutem Erfolg zur Bekämpfung von Plasmopara-Arten, wie beispielsweise Plasmopora viticola, an Reben und Phytophthora-Arten, wie beispielsweise Phytophthora infestans, an Tomaten eingesetzt werden. Besonders hervorzuheben ist, daß die erfindungsgemäßen Wirkstoffe nicht nur eine protektive Wirkung entfalten, sondern teilweise auch kurativ wirksam sind, also bei Anwendung nach der Kontamination mit den Sporen des Pilzes. Weiterhin ist auf die teilweise systemische Wirkung der Stoffe hinzuweisen. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und

Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.BV. durch Gießen, Tauchen Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Imkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele:

Herstellung des Ausgangsproduktes:

a)

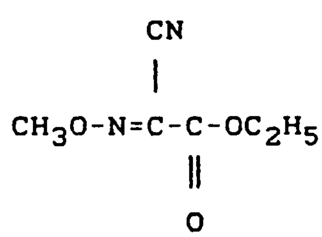

In eine Suspension von 164 g (1 Mol) 2-Cyan-2-hydroximinoessigsäureethylester,Natriumchlorid (G. Kinast, Libiegs Ann. Chem., 1981, 1561) und 138 g pulverisiertem Kaliumcarbonat in 1,5 l Aceton werden 161 g (1,25 Mol) Dimethylsulfat (98 %ig) in 30 Minuten getropft und das Reaktionsgemisch 3 Stunden unter Rückfluß erhitzt. Nach Abkühlen wird über Kieselgur filtriert und eingedampft.

Man erhält 124,8 g 2-Cyan-2-methoximino-essigsäure-ethylester (E-Isomeres) als rotbraunes Öl von 85 % Reinheit (GC). Die Ausbeute beträgt demnach 68 % der Theorie. Nach Chromatographie an der fünffachen Menge Kieselgel 60 mit Chloroform erhält man ein 93 %iges hellgelbes Öl

b)

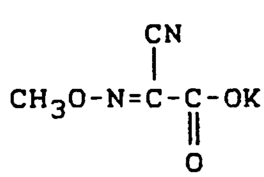

In eine Lösung von 124,8 g (0,672 Mol) 84 %igem 2-Cyan-2-methoximino-essigsäureethylesters (E-Isomeres) in 500 ml Ethanol wird bei 20° C eine Lösung von 45,1 g (0,806 Mol) Kaliumhydroxid in 500 ml Wasser getropft und das Reaktionsgemisch eine Stunde bei 40° C gerührt. Die Lösung wird im Vakuum bei 40° C eingedampft, der Rückstand mit Methanol 30 Minuten verrührt, abgesaugt, mit Ethanol, Acetonitril und Dichlormethan gewaschen und bei Raumtemperatur getrocknet.

Man erhält 58,6 g (53 % der Theorie) des Kaliumsalzes von 2-Cyan-2-methoximino-acetat (E-Isomeres).

c)

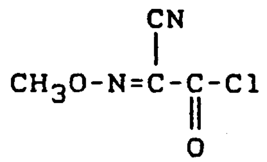

20 g (0,12 Mol) des Kaliumsalzes von 2-Cyan-2-methoximinoacetat (E-Isomeres) werden in 250 ml trockenem Ether suspendiert und nach Zugabe von einigen Tropfen Dimethylformamid bei 0° C tropfenweise mit 76,2 g (0,6 Mol) Oxalylchlorid versetzt. Das Reaktionsgemisch wird 2 Stunden bei 0° C gerührt, filtriert und das Filtrat im Vakuum bei Raumtemperatur eingedampft.

Man erhält 13,5 g (77 % der Theorie) 2-Cyan-2-methoximinoacetylchlorid (E-Isomeres) als gelbes Öl, das sofort weiter umgesetzt wird.

Beispiel 2

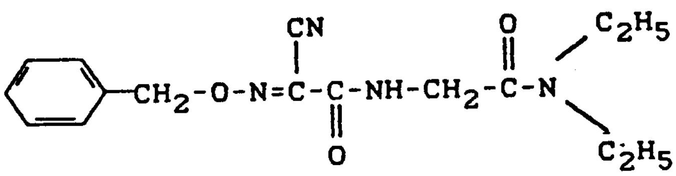

(Verfahren a)

In eine Lösung von 5,8 g (0,02 Mol) Glycindiethylamid, 5,05 g (0,05 Mol) Triethylamin und 0,61 g (0,005 Mol) 4-Dimethylaminopyridin in 75 ml Dichlormethan wird bei 0° C eine Lösung von 11,1 g (0,05 Mol) 2-Benzyloximino-2-cyanacetylchlorid (E-Isomeres) in 50 ml Dichlormethan getropft und das Reaktionsgemisch eine Stunde bei 0° C und 20 Stunden bei Raumtemperatur gerührt. Die Reaktionsmischung wird mit 50 ml Dichlormethan verdünnt, mit 1M Salzsäure, 10 %iger Natriumhydrogencarbonatlösung und Wasser gewaschen, über Magnesiumsulfat getrocknet und im Vakuum eingedampft. Der Rückstand wird aus Ligroin/Essigester umkristallisiert.

Man erhält 4,0 g (25 % der Theorie) $N^{\alpha}$-(2-Benzyloximino-2-cyan-acetyl)-glycindiethylamid (E-Isomeres) mit dem Schmelzpunkt 76-81° C.

Herstellung des Ausgangsproduktes:

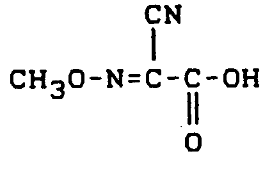

In eine Mischung aus 83 g (0,5 Mol) 2-Cyan-2-methoximinoessigsäureethylester (E-Isomeres) und 250 ml Wasser wird bei 20-30° C 550 ml (0,55 Mol) 1M Natronlauge getropft und das Reaktionsgemisch 3 1/2

Stunden bei Raumtemperatur gerührt. Die Lösung wird mit Ether gewaschen und auf eine Säule von 1 l Lewatit S 100 ($H^+$-Form, zuvor mit Aceton gewaschen) aufgetragen. Mit je 1 l Aceton und Acetonitril wird eluiert und das Eluat bei 30-40° C im Vakuum eingedampft. Man erhält 63,2 g (99 % der Theorie) 2-Cyan-2-methoximino-essigsäure (E-Isomeres) mit dem Schmelzpunkt 48-49° C.

Beispiel 4

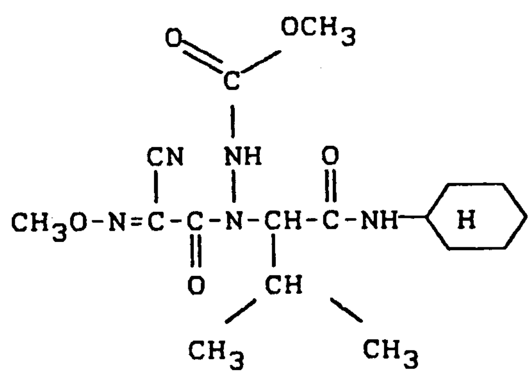

(Verfahren b)

In eine Lösung von 6,4 g (0,05 Mol) 2-Cyan-2-methoximino-essigsäure (E-Isomeres), 4,5 g (0,05 Mol) Carbazinsäuremethylester und 3,6 g (0,05 Mol) Isobutyraldehyd in 75 ml Methanol wird bei 0° C eine Lösung von 5,5 g (0,05 Mol) Cyclohexylisocyanid getropft und 24 Stunden bei Raumtemperatur gerührt. Das Lösungsmittel wird im Vakuum abdestilliert, der Rückstand zwischen je 200 ml Essigester und Wasser verteilt, die organische Phase mit je 150 ml 1M Salzsäure, 10 %iger Natriumhydrogencarbonatlösung und Wasser gewaschen, über Natriumsulfat getrocknet und im Vakuum eingeengt.

Man erhält 9,0 g (47 % der Theorie) $N^{\alpha}$-(2-(Cyan-2-methoximino-acetyl)-$N^{\alpha}$-methoxycarbonylamino-DL-valincyclohexylamid (E-Isomeres) mit dem Schmelzpunkt 132-134° C.

Beispiel 5

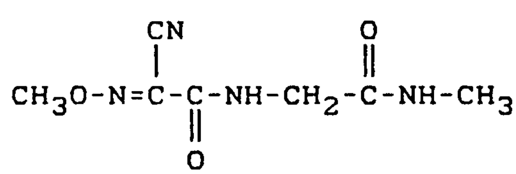

(Verfahren c)

Zu einer Lösung von 11,8 g (0,0553 Mol) N-(2-Cyan-2-methoximino-acetyl)-glycinethylester (E-Isomeres) in 50 ml Methanol werden bei 0° C 24,5 ml (0,166 Mol) einer 6,8M Lösung von Methylamin in Methanol getropft. Man läßt die Reaktionsmischung auf Raumtemperatur kommen und rührt noch eine Stunde bei 20° C. Nach Kühlung auf 10° C wird der kristalline Niederschlag abgesaugt, mit wenig kaltem Ethanol gewaschen und bei 40° C getrocknet.

Man erhält 7,0 g (64 % der Theorie) $N^{\alpha}$-(2-Cyan-2-methoximino-acetyl)-glycinmethylamid (E-Isomeres) mit dem Schmelzpunkt 140-141° C.

Beispiel 6

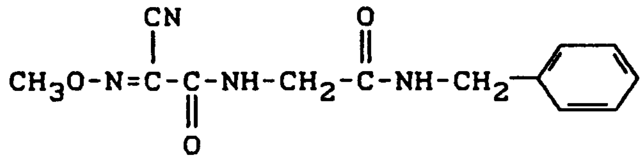

(Verfahren c)

Zu einer Lösung von 7,1 g (0,0356 Mol) $N^{\alpha}$-(2-Cyan-2-methoximino-acetyl)-glycinhydrazid (E-Isomeres) in 70 ml Dimethylformamid wird eine Lösung von 7,0 g (0,0712 Mol) konzentrierter Salzsäure in 10 ml Tetrahydrofuran gegeben und bei -20° C 5,15 g (0,042 Mol) Isoamylnitril (97 %ig) zugetropft. Nach 15 Minuten Rühren bei -20° C wird eine Lösung von 10,8 g (0,107 Mol) Triethylamin in 15 ml Dimethylformamid bei -20° C zugetropft und anschließend eine Lösung von 4,2 g (0,0392 Mol) Benzylamin in 3,5 ml Dimethylformamid hinzugegeben. Das Reaktionsgemisch wird 65 Stunden bei 0° C aufbewahrt, 24 Stunden bei 20° C gerührt und dann in 150 ml Wasser gegossen. Man extrahiert mit Essigester (2 mal je 150 ml), wäscht den Extrakt mit je 100 ml 1M Citronensäure, 10 %iger Natriumhydrogencarbonatlösung und Wasser, trocknet über Natriumsulfat und engt im Vakuum ein.

Man erhält 6,2 g (64 % der Theorie) $N^{\alpha}$-(2-Cyan-2-methoximino-acetyl)-glycinbenzylamid (E-Isomeres) mit dem Schmelzpunkt 101-102° C.

Herstellung des Ausgangsproduktes:

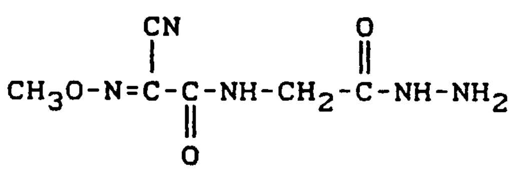

In eine Lösung von 30 g (0,155 Mol) N-(2-Cyan-2-methoximino-acetyl)-glycinethylester (E-Isomeres) in 150 ml Ethanol wird bei 24-30° C 15,5 g (0,31 Mol) Hydrazinhydrat getropft und das Reaktionsgemisch 4 Stunden bei Raumtemperaturgerührt. Nach Abkühlen auf 10° C wird der kristalline Niederschlag abgesaugt, mit 200 ml Ether gewaschen und bei Raumtemperatur getrocknet.

Man erhält 28,1 g (91 % der Theorie) $N^{\alpha}$-(2-Cyan-2-methoximino-acetyl)-glycinhydrazid (E-Isomeres) mit dem Schmelzpunkt 167-168° C.

In analoger Weise und entsprechend den erfindungsgemäßen Verfahren werden die nachfolgenden E-Isomeren der Verbindungen der allgemeinen Formel (I)

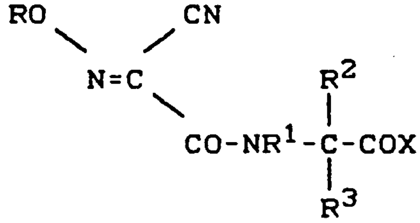

(I)

erhalten:

| Beisp.-Nr. | R | $R^1$ | $R^2$ | $R^3$ | X | Physikalische Konstante |
|---|---|---|---|---|---|---|
| 9 | $-CH_3$ | H | H | 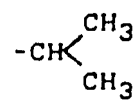 | 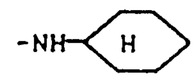 | Fp: 144-146°C |
| 10 | $-CH_3$ | 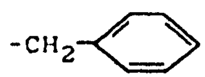 | H | H | 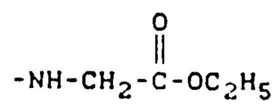 | $n_D^{20}$: 1,5122 |
| 11 | $-CH_3$ | H | H | H | 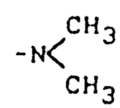 | Fp: 102-104°C |

| Beisp.-Nr. | R | $R^1$ | $R^2$ | $R^3$ | X | Physikalische Konstante |
|---|---|---|---|---|---|---|
| 15 | 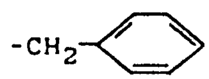 | $-CH_3$ | H | H | 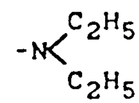 | $n_D^{20}$: 1,5190 |

| Beisp.-Nr. | R | $R^1$ | $R^2$ | $R^3$ | X | Physikalische Konstante |
|---|---|---|---|---|---|---|
| 20 | $-CH_2-$ 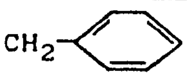 | H | H | $-CH_2-$ 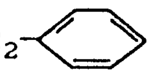 | $-NH-CH_3$ | Öl (L-Form) |
| 25 | $-CH_2-$ 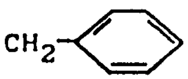 | H | H | 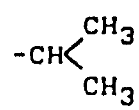 | $-NH-CH_3$ | Öl |
| 26 | $-CH_2-$ 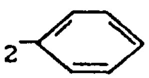 | H | H | H | $-NH-CH_3$ | Fp:122-125°C |

| Beisp.-Nr. | R | $R^1$ | $R^2$ | $R^3$ | X | Physikalische Konstante |
|---|---|---|---|---|---|---|
| 27 | 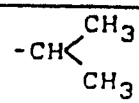 | H | H | H | $-NH-CH_3$ | Fp: 102-104°C |
| 29 | 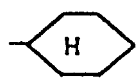 | H | H | H | $-NH-CH_3$ | Fp: 135-140°C |
| 30 | 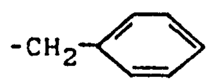 | H | H | $-CH_3$ | $-NH-CH_3$ | Fp: 177-181°C |
| 31 | 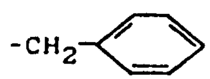 | H | H | H | 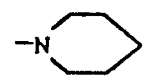 | Fp: 119-120°C |

| Beisp.-Nr. | R | $R^1$ | $R^2$ | $R^3$ | X | Physikalische Konstante |
|---|---|---|---|---|---|---|
| 33 | 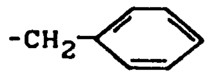 | H | H | H | 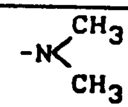 | Fp:132-138°C |
| 34 | 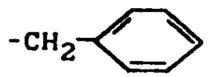 | H | H | H | 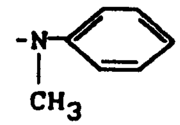 | Fp: 85-89 °C |
| 35 | 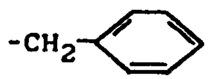 | H | H | H | 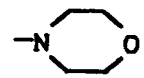 | Fp:127-128°C |
| 36 | 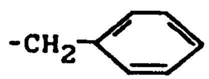 | H | H | H | 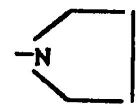 | Fp: 97-101°C |
| 37 | $-CH_3$ | H | H | H | 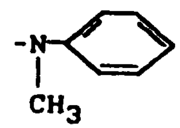 | Fp:154-155°C |
| 38 | $-CH_3$ | H | H | H | 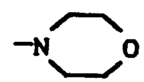 | Fp:172-173°C |

| Beisp.-Nr. | R | $R^1$ | $R^2$ | $R^3$ | X | Physikalische Konstante |
|---|---|---|---|---|---|---|
| 39 | $-CH_3$ | H | H | H | 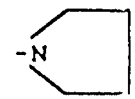 | Fp:101-105°C |
| 40 | $-CH_3$ | H | H | H | 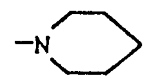 | Fp:137-139°C |
| 41 | $-CH_3$ | H | H | H | -OH | Fp: 95-100°C |
| 42 | $-CH_3$ | H | H | H | $-NH-C_2H_5$ | Fp:108-110°C |
| 45 | $-CH_3$ | H | H | H | $-N(C_2H_5)_2$ | Fp: 93-94 °G |

| Beisp.-Nr. | R | $R^1$ | $R^2$ | $R^3$ | X | Physikalische Konstante |
|---|---|---|---|---|---|---|
| 52 | $-CH_3$ | $-CH_3$ | H | H | $-N(C_2H_5)_2$ | $n_D^{23}$: 1,4857 |

| Beisp.-Nr. | R | $R^1$ | $R^2$ | $R^3$ | X | Physikalische Konstante |
|---|---|---|---|---|---|---|
| 56 | $-CH_3$ | H | H | H | -NH-N=CH- 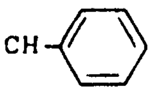 | Fp:204-206°C |
| 58 | $-CH_2$- 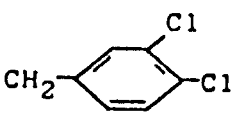 | H | H | H | $-NHCH_3$ | Fp:165-168°C |
| 59 | $-CH_2$- 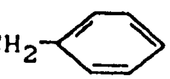 | H | H | $-CH(CH_3)_2$ | $-NHCH_3$ | Öl |
| 60 | $-CH_2$- 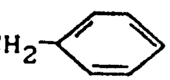 | H | H | $-CH_3$ | $-NHCH_3$ | Öl |

| Beisp.-Nr. | R | $R^1$ | $R^2$ | $R^3$ | X | Physikalische Konstante |
|---|---|---|---|---|---|---|
| 65 | $-CH_3$ | H | H | H | $-NH-CH_2-CH=CH_2$ | Fp: 85-88° C |

| Beisp.-Nr. | R | $R^1$ | $R^2$ | $R^3$ | X | Physikalische Konstante |
|---|---|---|---|---|---|---|
| 69 | $-C_2H_5$ | H | H | H | $-NH-CH_3$ | Fp: 144° C |
| 70 | $-CH_2-CH=CH_2$ | H | H | H | $-NH-CH_3$ | Fp: 133° C |
| 71 | $-CH_2-C \equiv CH$ | H | H | H | $-NH-CH_3$ | Fp: 148-150° C |
| 72 | $-CH_3$ | H | H | H | $-NH-CH_2-CO-OC_2H_5$ | Fp: 123-125° C |
| 73 | $-CH_3$ | H | H | $-CH(CH_3)_2$ | $-NH-CH_3$ | Fp: 130° C |
| 74 | $-CH_3$ | H | H | $-CH_2-$ 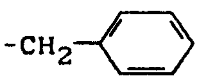 | $-NH-CH_3$ | Fp: 50° C |
| 75 | $-CH_3$ | H | H | $-CH(CH_3)_2$ | $-NH-CH_3$ | Fp: 130° C |
| 76 | $-CH_2-$ 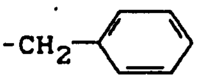 | H | H | $-CH_3$ | $-NH-NH-CO-NH-CH_3$ | Fp: 178° C (L-Form) |
| 77 | $-CH_2-$ 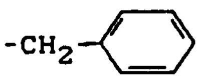 | H | H | $-CH_3$ | $-NH-NH-CO-NH-CH_3$ | Fp: 135° C (DL-Form) |
| 78 | $-CH_2-$ 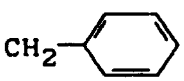 | H | H | $-CH_2-OH$ | $-NH-NH-CO-NH-CH_3$ | Fp: 219° C |

| Beisp.-Nr. | R | $R^1$ | $R^2$ | $R^3$ | X | Physikalische Konstante |
|---|---|---|---|---|---|---|
| 80 | $-CH_3$ | H | H | 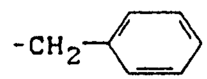 | $-NH-CH_3$ | Fp: 198-200° C |
| 81 | $-CH_3$ | H | H | 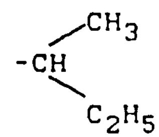 | $-NH-CH_3$ | Fp: 118-120° C |
| 82 | $-CH_3$ | H | H | H | $-NH-CH_2-CO-NH-CH_3$ | Fp: 212-214° C |
| 84 | $-CH_3$ | H | H | $-CH_2-CH(CH_3)_2$ | $-NH-CH_3$ | Öl |
| 85 | $-CH_3$ | H | H | 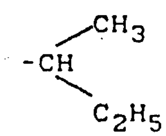 | $-NH-CH_3$ | Fp: 125-126° C |

| Beisp.-Nr. | R | $R^1$ | $R^2$ | $R^3$ | X | Physikalische Konstante |
|---|---|---|---|---|---|---|
| 86 | $-CH_3$ | H | $CH_2$ | $CH_2$ | $-NH-CH_3$ | Fp: 98-100° C |
| 87 | $-CH_3$ | H | H | $-CH_2-$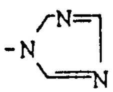 | $-NH-CH_3$ | Fp: 151-152° C |
| 88 | $-CH_3$ | H | H | $-CH_3$ | $-NH-CH_3$ | Fp: 157-158° C |
| 90 | $-CH_3$ | H | H | 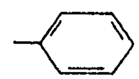 | $-NH-CH_3$ | Öl |
| 91 | $-CH_3$ | H | H | 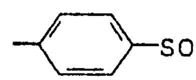$-SO_2-CH_3$ | $-NH-CH_3$ | Fp: 211-212° C |
| 92 | $-CH_3$ | H | H | H | $-NH-C(CH_3)_3$ | Fp: 152-153° C |
| 93 | $-CH_3$ | H | H | H | $-NH-CH_2-CH_2-N(C_2H_5)_2$ | Öl |
| 94 | $-CH_3$ | H | H | H | $-NH-CH(CH_3)_2$ | Fp: 132-133° C |

| Beisp.-Nr. | R | $R^1$ | $R^2$ | $R^3$ | X | Physikalische Konstante |
|---|---|---|---|---|---|---|
| 95 | $-CH_3$ | H | H | H | $-NH-CH_2-CH_2-$ 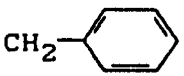 | Fp: 92-95° C |
| 96 | $-CH_3$ | H | H | H | $-NH-CH-$ 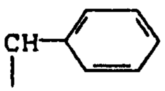<br>$CH_3$ | Fp: 96-98° C |
| 97 | $-CH_3$ | H | H | H | $-NH-CH_2-CH_2-Cl$ | Fp: 91-94° C |
| 98 | $-CH_3$ | H | H | H | $-NH-CH(CH_3)_2$ | Fp: 86-88° C |
| 99 | $-CH_3$ | H | H | H | $-NH-C(CH_3)_3$ | Fp: 80-81° C |
| 100 | $-CH_3$ | H | H | H | $-NH-CH_2-CH(CH_3)_2$ | Fp: 100-102° C |
| 101 | $-CH_3$ | H | H | H | $CH_3$<br>$-NH-CH-CH_2-CH_3$ | Fp: 111-112° C |
| 102 | $-CH_3$ | H | H | H | $-NH-CH_2-CH_2-O-CH_3$ | Fp: 102-105° C |
| 103 | $-CH_3$ | H | H | H | $-NH-CH_3$ x 1/2 $CuCl_2$ | Fp: 160-165° C |

Verwendungsbeispiele

In den nachfolgenden Verwendungsbeispielen werden die nachfolgend angegebenen Substanzen als Vergleichsverbindungen eingesetzt:

(A)

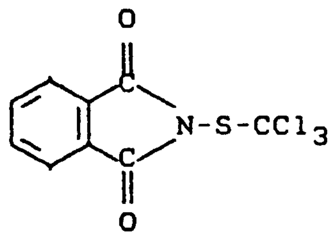

(B)

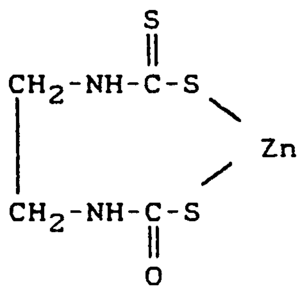

(C)

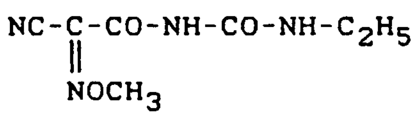

(US 3.957.847)

Beispiel A

Plasmopara-Test (Reben) / protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22° C und 100 % relativer Luftfeuchtigkeit. Anschließend werden die Pflanzen 5 Tage im Gewächshaus bei 22° C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigt in diesem Test z.B. die Verbindung gemäß dem Herstellungsbeispiel 5.

Beispiel B

Phytophthora-Test (Tomate) /kurativ

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20° C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100 % relativer Luftfeuchtigkeit und ca. 20° C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß Herstellungsbeispielen: 1 und 5.

Beispiel C

Phytophthora-Test (Tomate)/systemisch

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften wird die Wirkstoffzubereitung auf Einheitserde gegossen, in der sich junge versuchsbereite Pflanzen befinden. 3 Tage nach der Behandlung werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20° C aufgestellt.

3 Tage nach der Inokulation erfolgt die Auswertung.

Eine deutliche Überlegenheit in der Wirksamkeit gegenüber dem Stand der Technik zeigt in diesem Test z.B. die Verbindung gemäß dem Herstellungsbeispiel 5.

**Patentansprüche**

**1.** E-Isomere von $N^{\alpha}$-(2-Cyan-2-alkoximino-acetyl)-aminosäurederivaten und -peptiden der allgemeinem Formel (I)

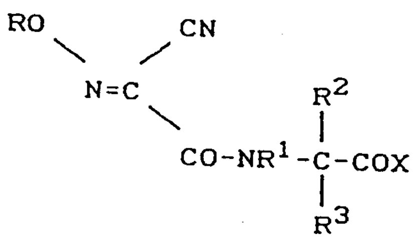

(I)

in welcher

R für geradkettiges oder verzweigtes Alkyl mit 1 bis 8 Kohlenstoffatomen, Alkenyl oder Alkinyl mit jeweils 2 bis 4 Kohlenstoffatomen, für Cyanalkyl mit 1 bis 4 Kohlenstoffatomen, für gegebenenfalls im Phenylteil einfach bis dreifach, gleich oder verschieden substituiertes Phenyl-alkyl mit 1 bis 4 Kohlenstoffatomen im Alkylteil steht, wobei als

Substituenten genannt seien: Halogen, Cyano, Nitro, Hydroxy, Alkyl, Alkoxy, Alkylthio, Alkylsulfinyl und Alkylsulfonyl mit jeweils 1 bis 4 Kohlenstoffatomen und gegebenenfalls einfach bis dreifach, gleich oder verschieden durch Halogen substituiertes Phenyl, oder für gegebenenfalls einfach bis dreifach, gleich oder verschieden substituiertes Cycloalkyl mit 3 bis 7 Kohlenstoffatomen steht, wobei als Substituenten Halogen und Alkyl mit 1 bis 4 Kohlenstoffatomen genannt seien;

$R^1$ für Wasserstoff, geradkettiges oder verzweigtes Alkyl mit 1 bis 4 Kohlenstoffatomen, für jeweils gegebenenfalls einfach bis dreifach, gleich oder verschieden substituiertes Phenyl oder Benzyl steht, wobei als Substituenten die bei R bereits genannten Phenylsubstituenten in Frage kommen, oder für Alkoxycarbonylamino mit 1 bis 4 Kohlenstoffatomen im Alkylteil steht;

$R^2$ für Wasserstoff oder geradkettiges oder verzweigtes Alkyl mit 1 bis 4 Kohlenstoffatomen steht;

$R^3$ für Wasserstoff, geradkettiges oder verzweigtes Alkyl mit 1 bis 6 Kohlenstoffatomen, für geradkettiges oder verzweigtes Hydroxyalkyl mit 1 bis 4 Kohlenstoffatomen, für Alkoxycarbonylalkyl mit jeweils 1 bis 4 Kohlenstoffatomen im Alkoxyteil und im Alkylteil, für Hydroxycarbonylalkyl und Aminocarbonylalkyl mit jeweils 1 bis 4 Kohlenstoffatomen im Alkylteil, für Cyanalkyl mit 1 bis 4 Kohlenstoffatomen, für Alkenyl oder Alkinyl mit jeweils 2 bis 4 Kohlenstoffatomen, für gegebenenfalls einfach bis dreifach, gleich oder verschieden substituiertes Cycloalkyl mit 3 bis 7 Kohlenstoffatomen steht, wobei als Substituenten Halogen und Alkyl mit 1 bis 4 Kohlenstoffatomen genannt seien, ferner für gegebenenfalls im Phenylteil einfach bis dreifach, gleich oder verschieden substituiertes Phenyl oder Phenylalkyl mit 1 bis 4 Kohlenstoffatomen im Alkylteil steht, wobei als Substituenten jeweils die bei R bereits genannten Phenylsubstituenten genannt seien, oder für die Gruppierung

$R^4$-$SO_n$-Z-

steht, wobei

$R^4$ für Wasserstoff, geradkettiges oder verzweigtes Alkyl mit 1 bis 4 Kohlenstoffatomen, für gegebenenfalls im Phenylteil einfach bis dreifach, gleich oder verschieden substituiertes Phenylalkyl mit 1 bis 4 Kohlenstoffatomen im Alkylteil steht, wobei als Substituenten die bei R bereits genannten Phenylsubstituenten genannt seien,

n für die Zahlen 0, 1 oder 2 steht und

Z für eine geradkettige oder verzweigte Alkylenkette mit 1 bis 4 Kohlenstoffatomen steht;

$R^2$ und $R^3$ gemeinsam mit dem Kohlenstoffatom, an das gebunden sind, für Cycloalkyliden mit 3 bis 6 Kohlenstoffatomen stehen;

X für die Gruppierungen $-OR^I$ oder $-NR^{II}R^{III}$ steht, wobei

$R^I$ für Wasserstoff,

$R^{II}$ für Wasserstoff oder geradkettiges oder verzweigtes Alkyl mit 1 bis 4 Kohlenstoffatomen steht;

$R^{III}$ für geradkettiges oder verzweigtes Alkyl mit 1 bis 4 Kohlenstoffatomen, für Alkenyl mit 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit bis zu 4 Kohlenstoffatomen in den einzelnen Alkylteilen, für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil, für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei R bereits genannten Phenylsubstituenten in Frage kommen, für gegebenenfalls gleich oder verschieden substituiertes Cycloalkyl mit 3 bis 7 Kohlenstoffatomen, wobei als Substituenten Halogen und Alkyl mit 1 bis 4 Kohlenstoffatomen in Frage kommen, für Alkylcarbonylamino oder Alkoxycarbonylamino mit jeweils 1 bis 4 Kohlenstoffatomen im Alkylteil

und im Alkoxyteil, für Aminocarbonylamino, Alkylaminocarbonylamino und Dialkylaminocarbonylamino mit jeweils 1 bis 4 Kohlenstoffatomen in den einzelnen Alkylteilen sowie für Formylamino oder für die Gruppierung

$-N=CH-R^{IV}$

steht, wobei

$R^{IV}$ für geradkettiges oder verzweigtes Alkyl mit 1 bis 4 Kohlenstoffatomen oder gegebenenfalls einfach bis dreifach, gleich oder verschieden substituiertes Phenyl steht, wobei als Substituenten die bei R bereits genannten Phenylsubstituenten in Frage kommen,

oder

$R^{II}$ und $R^{III}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann, und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl sowie Alkylaminocarbonyl und Dialkylaminocarbonyl mit jeweils 1 bis 4 Kohlenstoffatomen in den einzelnen Alkylteilen substituiert sein kann, sowie deren physiologisch verträglichen gegebenenfalls substituierten Ammonium-, Alkali- und Erdalkali-Salze und Metallsalz-Komplexe,

ausgenommen Verbindungen,
in denen $R^{III}$ für Alkyl steht,
wenn $R^1$ und $R^{II}$ für Wasserstoff stehen.

**2.** E-Isomere von $N^{\alpha}$-(2-Cyan-2-alkoximino-acetyl-aminosäurederivaten und -peptiden der Formel (I) gemäß Anspruch 1, in welcher

R für geradkettiges oder verzweigtes Alkyl mit 1 bis 4 Kohlenstoffatomen, Allyl, Propargyl, Cyanmethyl, Cyanethyl, für gegebenenfalls im Phenylteil einfach oder zweifach, gleich oder verschieden substituiertes Phenylalkyl mit 1 oder 2 Kohlenstoffatomen im Alkylteil steht, wobei als Substituenten genannt seien: Fluor, Chlor, Cyano, Nitro, Hydroxy, Methyl, Methoxy, Methylthio, Methylsulfinyl, Methylsulfonyl und gegebenenfalls einfach oder zweifach, gleich oder verschieden durch Fluor oder Chlor substituiertes Phenyl; ferner für jeweils gegebenenfalls einfach oder zweifach, gleich oder verschieden durch Fluor, Chlor oder Methyl substituiertes Cyclopropyl, Cyclobutyl, Cyclopentyl, Cylohexyl oder Cycloheptyl steht;

$R^1$ für Wasserstoff, Methyl, Ethyl, für jeweils gegebenenfalls einfach oder zweifach, gleich oder verschieden substituiertes Phenyl oder Benzyl steht, wobei als Substituenten insbesondere die bei R bereits genannten Phenylsubstituenten in Frage kommen; ferner für Methoxycarbonylamino oder Ethoxycarbonylamino steht;

$R^2$ für Wasserstoff, Methyl oder Ethyl steht;

$R^3$ für Wasserstoff, geradkettiges oder verzweigtes Alkyl mit 1 bis 4 Kohlenstoffatomen, 1-Hydroxymethyl, Hydroxyethyl, für Methoxycarbonylmethyl, Methoxycarbonylethyl, Ethoxycarbonylethyl, Hydroxycarbonylmethyl, Hydroycarbonylethyl, Aminocarbonylmethyl, Amonocarbonylethyl, für Cyanmethyl, Cyanethyl, für Allyl, für Propargyl, für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht; ferner für gegebenenfalls im Phenylteil einfach oder zweifach, gleich oder verschieden substituiertes Phenylalkyl mit 1 oder 2 Kohlenstoffatomen im Alkylteil oder einfach oder zweifach, gleich oder verschieden substituiertes Phenyl steht, wobei als Substituenten die bei R bereits genannten Phenylsubstituenten in Frage kommen, ferner für die Gruppierung

$R^4-SO_n-Z-$

steht, wobei

$R^4$ für Wasserstoff, Methyl, Ethyl oder für gegebenenfalls im Phenylteil einfach oder zweifach, gleich oder verschieden substituiertes Phenylalkyl mit 1 oder 2 Kohlenstoffatomen im Alkylteil steht, wobei als Substituenten insbesondere die bei R bereits

genannten Phenylsubstituenten in Frage kommen,

n für die Zahlen 0, 1 oder 2 steht und

Z für eine geradkettige oder verzweigte Alkylenkette mit 1 oder 2 Kohlenstoffatomen steht;

$R^2$ und $R^3$ gemeinsam mit dem Kohlenstoffatom, an das sie gebunden sind, für Cyclopropyliden stehen, und

X für die Gruppierungen $-OR^I$ oder $-NR^{II}R^{III}$ steht, wobei

$R^1$ für Wasserstoff;

$R^{II}$ für Wasserstoff, Methyl oder Ethyl steht;

$R^{III}$ für Methyl, Ethyl, n-Propyl,i-Propyl, n-Butyl, i-Butyl, sec.-Butyl, tert.-Butyl, Allyl, für Halogenalkyl mit 1 oder 2 Kohlenstoffatomen und 1 bis 3 gleichen oder verschiedenen Halogenatomen, wie Fluor- und Chloratomen, für Alkoxyalkyl mit jeweils 1 oder 2 Kohlenstoffatomen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanoalkyl mit 1 oder 2 Kohlenstoffatomen im Alkylteil, für gegebenenfalls im Phenylteil einfach oder zweifach, gleich oder verschieden substituiertes Phenylalkyl mit 1 oder 2 Kohlenstoffatomen im Alkylteil, für einfach oder zweifach, gleich oder verschieden substituiertes Phenyl steht, wobei als Substituenten die bei R bereits genannten Phenylsubstituenten in Frage kommen; für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl), für Alkylcarbonylamino oder Alkoxycarbonylamino mit jeweils 1 oder 2 Kohlenstoffatomen im Alkylteil und im Alkoxyteil, für Aminocarbonylamino, Methylaminocarbonylamino, Ethylaminocarbonylamino, Dimethylaminocarbonylamino, Diethylaminocarbonylamino, Methylethylaminocarbonylamino sowie für Formylamino oder für die Gruppierung

$-N=CH-R^{IV}$

steht, wobei

$R^{IV}$ für Methyl, Ethyl, für gegebenenfalls einoder zweifach, gleich oder verschieden substituiertes Phenyl steht, wobei als Substituenten die bei R genannten Phenylsubstituenten in Frage kommen; oder

$R^{II}$ und $R^{III}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann und gegebenenfalls durch Cyano, Fluor, Chlor, Brom, Iod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl sowie Methylethylaminocarbonyl substituiert sein kann und deren physiologisch verträglichen gegebenenfalls substituierten Ammonium-, Alkali- und Erdalkali-Salze und Metallsalz-Komplexe,

ausgenommen Verbindungen in denen $R^{III}$ für Alkyl steht wenn $R^1$ und $R^{II}$ für Wasserstoff stehen.

**3.** Verfahren zur Herstellung von E-Isomeren von $N^\alpha$-(2-Cyan-2-alkoximinoacetyl)-aminosäurederivaten und -peptiden der Formel (I)

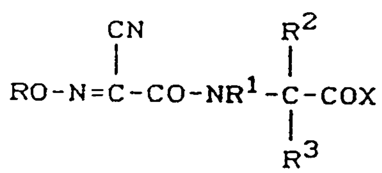

(I)

in welcher

R für geradkettiges oder verzweigtes Alkyl mit 1 bis 8 Kohlenstoffatomen, Alkenyl oder Alkinyl mit jeweils 2 bis 4 Kohlenstoffatomen, für Cyanalkyl mit 1 bis 4 Kohlenstoffatomen, für gegebenenfalls im Phenylteil einfach bis dreifach, gleich oder verschieden

substituiertes Phenyl-alkyl mit 1 bis 4 Kohlenstoffatomen im Alkylteil steht, wobei als Substituenten genannt seien: Halogen, Cyano, Nitro, Hydroxy, Alkyl, Alkoxy, Alkylthio, Alkylsulfinyl und Alkylsulfonyl mit jeweils 1 bis 4 Kohlenstoffatomen und gegebenenfalls einfach bis dreifach, gleich oder verschieden durch Halogen substituiertes Phenyl, oder für gegebenenfalls einfach bis dreifach, gleich oder verschieden substituiertes Cycloalkyl mit 3 bis 7 Kohlenstoffatomen steht, wobei als Substituenten Halogen und Alkyl mit 1 bis 4 Kohlenstoffatomen genannt seien;

$R^1$ für Wasserstoff, geradkettiges oder verzweigtes Alkyl mit 1 bis 4 Kohlenstoffatomen, für jeweils gegebenenfalls einfach bis dreifach, gleich oder verschieden substituiertes Phenyl oder Benzyl steht, wobei als Substituenten die bei R bereits genannten Phenylsubstituenten in Frage kommen, oder für Alkoxycarbonylamino mit 1 bis 4 Kohlenstoffatomen im Alkylteil steht;

$R^2$ für Wasserstoff oder geradkettiges oder verzweigtes Alkyl mit 1 bis 4 Kohlenstoffatomen steht;

$R^3$ für Wasserstoff, geradkettiges oder verzweigtes Alkyl mit 1 bis 6 Kohlenstoffatomen, für geradkettiges oder verzweigtes Hydroxyalkyl mit 1 bis 4 Kohlenstoffatomen, für Alkoxycarbonylalkyl mit jeweils 1 bis 4 Kohlenstoffatomen im Alkoxyteil und im Alkylteil, für Hydroxycarbonylalkyl und Aminocarbonylalkyl mit jeweils 1 bis 4 Kohlenstoffatomen im Alkylteil, für Cyanalkyl mit 1 bis 4 Kohlenstoffatomen, für Alkenyl oder Alkinyl mit jeweils 2 bis 4 Kohlenstoffatomen, für gegebenenfalls einfach bis dreifach, gleich oder verschieden substituiertes Cycloalkyl mit 3 bis 7 Kohlenstoffatomen steht, wobei als Substituenten Halogen und Alkyl mit 1 bis 4 Kohlenstoffatomen genannt seien, ferner für gegebenenfalls im Phenylteil einfach bis dreifach, gleich oder verschieden substituiertes Phenyl oder Phenylalkyl mit 1 bis 4 Kohlenstoffatomen im Alkylteil steht, wobei als Substituenten jeweils die bei R bereits genannten Phenylsubstituenten genannt seien, oder für die Gruppierung

$R^4\text{-}SO_n\text{-}Z\text{-}$

steht, wobei

$R^4$ für Wasserstoff, geradkettiges oder verzweigtes Alkyl mit 1 bis 4 Kohlenstoffatomen, für gegebenenfalls im Phenylteil einfach bis dreifach, gleich oder verschieden substituiertes Phenylalkyl mit 1 bis 4 Kohlenstoffatomen im Alkylteil steht, wobei als Substituenten die bei R bereits genannten Phenylsubstituenten genannt seien,

n für die Zahlen 0, 1 oder 2 steht und

Z für eine geradkettige oder verzweigte Alkylenkette mit 1 bis 4 Kohlenstoffatomen steht;

$R^2$ und $R^3$ gemeinsam mit dem Kohlenstoffatom, an das gebunden sind, für Cycloalkyliden mit 3 bis 6 Kohlenstoffatomen stehen;

X für die Gruppierungen $-OR^I$ oder $-NR^{II}R^{III}$ steht, wobei

$R^I$ für Wasserstoff,

$R^{II}$ für Wasserstoff oder geradkettiges oder verzweigtes Alkyl mit 1 bis 4 Kohlenstoffatomen steht;

$R^{III}$ für geradkettiges oder verzweigtes Alkyl mit 1 bis 4 Kohlenstoffatomen, für Alkenyl mit jeweils 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit bis zu 4 Kohlenstoffatomen in den einzelnen Alkylteilen, für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil, für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei R bereits genannten Phenylsubstituenten in Frage kommen, für gegebenenfalls gleich oder verschieden substituiertes Cycloalkyl mit 3 bis 7 Kohlenstoffatomen, wobei als Substituenten Halogen und Alkyl mit 1 bis 4 Kohlenstoffatomen in Frage kommen, für Alkylcarbonylamino oder Alkoxycarbonylamino mit jeweils 1 bis 4 Kohlenstoffatomen im Alkylteil und im Alkoxyteil, für Aminocarbonylamino, Alkylaminocarbonylamino und Dialkylaminocarbonylamino mit jeweils 1 bis 4 Kohlenstoffatomen in den einzelnen Alkylteilen sowie für Formylamino oder für die Gruppierung

$-N = CH-R^{IV}$

steht, wobei

$R^{IV}$ für geradkettiges oder verzweigtes Alkyl mit 1 bis 4 Kohlenstoffatomen oder gegebenenfalls einfach bis dreifach, gleich oder verschieden substituiertes Phenyl steht, wobei als Substituenten die bei R bereits genannten Phenylsubstituenten in Frage kommen,

oder

$R^{II}$ und $R^{III}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann, und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl sowie Alkylaminocarbonyl und Dialkylaminocarbonyl mit jeweils 1 bis 4 Kohlenstoffatomen in den einzelnen Alkylteilen substituiert sein kann, sowie deren physiologisch verträglichen gegebenenfalls substituierten Ammonium-, Alkali- und Erdalkali-Salze und Metallsalz-Komplexe,

ausgenommen die Verbindungen,
in denen $R^{III}$ für Alkyl steht,
wenn $R^{II}$ und $R^1$ für Wasserstoff stehen,
dadurch gekennzeichnet, daß man

(a) Aminosäure-Derivate der Formel (II)

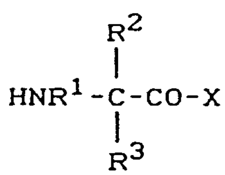

(II)

in welcher $R^1$, $R^2$, $R^3$ und X die oben angegebene Bedeutung haben, mit den carboxy-aktivierten Derivaten der E-Isomeren der Carbonsäuren der Formel (III)

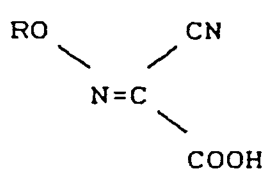

(III)

in welcher R die oben angegebene Bedeutung hat, gegebenenfalls in Gegenwart eines Katalysators, gegebenenfalls in Gegenwart eines Säurebindemittels und gegebenenfalls in Gegenwart eines Verdünnungsmittels umsetzt,

oder

(b) carboxy-aktivierte Derivate der E-Isomeren von Carbonsäuren der Formel (III)

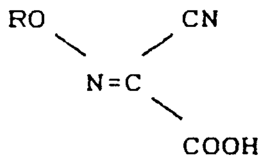

(III)

in welcher R die oben angegebene Bedeutung hat, zunächst mit Aminen der Formel (IV)

$H_2NR^1$ (IV)

in welcher $R^1$ die oben angegebene Bedeutung hat, anschließend mit Carbonyl-Derivaten der Formel (V)

$R^2$-CO-$R^3$ (V)

in welcher $R^2$ und $R^3$ die oben angegebenen Bedeutungen haben,
und schließlich mit Isonitrilen der Formel (VI)

CN-$R^5$ (VI)

in welcher
$R^5$ für Alkyl, Alkenyl, Alkinyl, Cyanoalkyl, Alkoxycarbonylalkyl oder für gegebenenfalls substituiertes Cycloalkyl, Phenyl oder Phenylalkyl steht,
gegebenenfalls in Gegenwart eines Verdünnungsmittels umsetzt, oder
(c) carboxy-aktivierte Derivate der E-Isomeren von Aminosäuren der Formel (Ia)

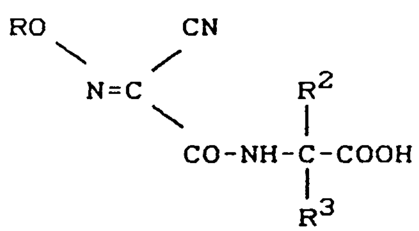

(Ia)

in welcher
R, $R^2$ und $R^3$ die oben angegebenen Bedeutungen haben,
mit Aminen der Formel (VII)

H-$NR^{II}R^{III}$ (VII)

in welcher
$R^{II}$ und $R^{III}$ die oben angegebenen Bedeutungen haben, gegebenenfalls in Gegenwart eines Katalysators und gegebenenfalls in Gegenwart eines Säurebindemittels und gegebenenfalls in Gegenwart eines Verdünnungsmittels umsetzt,
oder

(d) E-Isomere von Aminosäurederivaten der Formel (Ib)

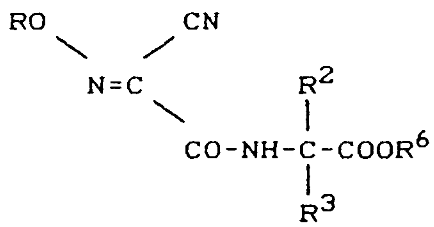

(Ib)

in welcher

R, $R^2$ und $R^3$ die oben angegebenen Bedeutungen haben, und

$R^6$ für Alkyl mit 1 bis 4 Kohlenstoffatomen steht, mit Aminen der Formel (VII)

H-NR''R''' (VII)

in welcher

R'' und R''' die oben angegebenen Bedeutungen haben,

gegebenenfalls in Gegenwart eines Verdünnungsmittels umsetzt und gegebenenfalls anschließend zu einem gegebenenfalls substituierten Ammonium-, Alkali- oder Erdalkali-Salz oder einen Metallsalz-Komplex umsetzt.

**4.** Schädlingsbekämpfungsmittel, gekennzeichnet durch einen Gehalt an mindestens einem E-Isomeren von $N^{\alpha}$-(2-Cyan-2-alkoximino-acetyl)-aminosäurederivat und -peptid der Formel (I) nach den Ansprüchen 1 bis 3.

**5.** Verwendung von E-Isomeren von $N^{\alpha}$-(2-Cyan-2-alkoximinoacetyl)-aminosäurederivaten und -peptiden der Formel (I) nach den Ansprüchen 1 bis 3 zur Bekämpfung von Schädlingen.

**6.** Verfahren zur Bekämpfung von Schädlingen, dadurch gekennzeichnet, daß man E-Isomere von $N^{\alpha}$-(2-Cyan-2-alkoximino-acetyl)-aminosäurederivaten und -peptiden der Formel (I) nach den Ansprüchen 1 bis 3 auf Schädlinge und/oder ihren Lebensraum einwirken läßt.

**7.** Verfahren zur Herstellung von Schädlingsbekämpfungsmitteln, dadurch gekennzeichnet, daß man E-Isomere von $N^{\alpha}$-(2-Cyan-2-alkoximinoacetyl)-aminosäurederivaten und -peptiden der Formel (I) nach den Ansprüchen 1 bis 3 mit Streckmitteln und/oder oberflächenaktiven Mitteln vermischt.

**8.** Verwendung von E-Isomeren von $N^{\alpha}$-(2-Cyan-2-alkoximinoaceytl)aminosäurederivaten und -peptiden der Formel (I) gemäß Anspruch 5 zur Bekämpfung von Pilzen.

**9.** E-Isomere von $N^{\alpha}$-(2-Cyan-2-alkoximino-acetyl)-aminosäurederivaten und -peptiden dadurch gekennzeichnet, daß in der Formel (XI)

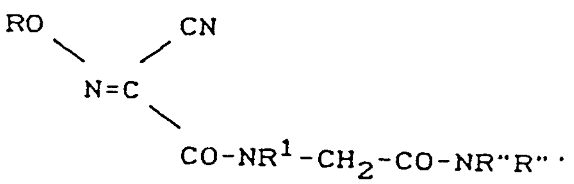

(XI)

a)

R für Methyl oder Benzyl,

$R^1$ für Wasserstoff und

R'' gleich R''' für Methyl oder Ethyl steht, oder

b)

| | |
|---|---|
| R | für Methyl oder Benzyl, |
| $R^1$ | für Methyl und |
| R'' gleich R''' | für Ethyl steht. |

**10.** E-Isomere von $N^\alpha$-(2-Cyan-2-alkoximino-acetyl)-aminosäurederivaten und -peptiden dadurch gekennzeichnet, daß in der Formel (XII)

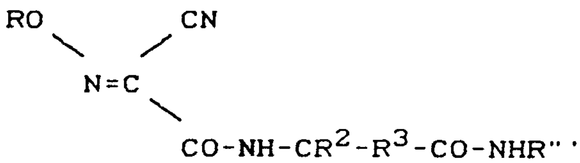

(XII)

a)

| | |
|---|---|
| R | für Methyl, Benzyl, 2-Propyl, Cyclohexyl, 3,4-Dichlorbenzyl, Ethyl, 2-Propenyl oder 2-Propinyl, |
| $R^2$, $R^3$ | für Wasserstoff und |
| R''' | für Methyl steht, oder |

b)

| | |
|---|---|
| R | für Benzyl, |
| $R^2$ | für Wasserstoff, |
| $R^3$ | für Methyl, 2-Propyl oder Benzyl und |
| R''' | für Methyl steht, oder |

c)

| | |
|---|---|
| R | für Methyl, |
| $R^2$ | für Methyl, |
| $R^3$ | für Wasserstoff und |
| R''' | für Methyl steht, oder |

d)

| | |
|---|---|
| R | für Methyl, |
| $R^2$, $R^3$ | für Wasserstoff und |
| R''' | für Benzyl, Ethyl, 2-Propenyl, t-Butyl, 2-Propyl, 2-Phenylethyl, i- oder s-Butyl steht. |

## Claims

**1.** E-Isomers of $N^\alpha$-(2-cyano-2-alkoximino-acetyl)-amino acid derivatives and peptides of the general formula (I)

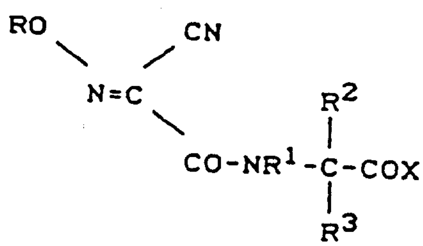

(I)

in which

R represents straight-chain or branched alkyl with 1 to 8 carbon atoms, or alkenyl or alkinyl with in each case 2 to 4 carbon atoms, or represents cyanoalkyl with 1 to 4 carbon atoms, or represents phenyl-alkyl which has 1 to 4 carbon atoms in the alkyl part and is optionally mono-, di- or trisubstituted in the phenyl part by identical or different substituents, substitutents which may be mentioned being: halogen, cyano, nitro, hydroxyl, alkyl, alkoxy, alkylthio, alkylsulphinyl and alkylsulphonyl with in each

case 1 to 4 carbon atoms and phenyl which is optionally mono-, di- or tri-substituted by identical or different halogen substituents, or represents cycloalkyl which has 3 to 7 carbon atoms and is optionally mono-, di- or trisubstituted by identical or different substituents, substituents which may be mentioned being halogen and alkyl with 1 to 4 carbon atoms;

$R^1$ represents hydrogen, or straight-chain or branched alkyl with 1 to 4 carbon atoms, or represents phenyl or benzyl, in each case optionally mono-, di- or trisubstituted by identical or different substituents, possible substituents being the phenyl substituents already mentioned for R, or represents alkoxycarbonylamino with 1 to 4 carbon atoms in the alkyl part,

$R^2$ represents hydrogen, or straight-chain or branched alkyl with 1 to 4 carbon atoms;

$R^3$ represents hydrogen, or straight-chain or branched alkyl with 1 to 6 carbon atoms, or represents straight-chain or branched hydroxyalkyl with 1 to 4 carbon atoms, or represents alkoxycarbonylalkyl with in each case 1 to 4 carbon atoms in the alkoxy part and in the alkyl part, or represents hydroxycarbonylalkyl and aminocarbonylalkyl with in each case 1 to 4 carbon atoms in the alkyl part, or represents cyanoalkyl with 1 to 4 carbon atoms, or represents alkenyl or alkinyl with in each case 2 to 4 carbon atoms, or represents cycloalkyl which has 3 to 7 carbon atoms and is optionally mono-, di- or trisubstituted by identical or different substituents, substituents which may be mentioned being halogen and alkyl with 1 to 4 carbon atoms, or furthermore represents phenyl or phenylalkyl, with 1 to 4 carbon atoms in the alkyl part, optionally mono- or di- or trisubstituted in the phenyl part by identical or different substituents, substituents which may be mentioned in each case being the phenyl substutuents already mentioned for R, or represents the grouping

$R^4$-$SO_n$-Z- ,

wherein

$R^4$ represents hydrogen, or straight-chain or branched alkyl with 1 to 4 carbon atoms, or represents phenylalkyl which has 1 to 4 carbon atoms in the alkyl part and is optionally mono-, di- or trisubstituted in the phenyl part by identical or different substituents, possible substituents being the phenyl substituents already mentioned for R,

n represents the number 0, 1 or 2 and

Z represents a straight-chain or branched alkylene chain with 1 to 4 carbon atoms;

$R^2$ and $R^3$, together with the carbon atom to which they are bonded, represent cycloalkylidene with 3 to 6 carbon atoms;

X represents the groupings $-OR^I$ or $-NR^{II}R^{III}$, wherein

$R^I$ represents hydrogen,

$R^{II}$ represents hydrogen, or straight-chain or branched alkyl with 1 to 4 carbon atoms;

$R^{III}$ represents straight-chain or branched alkyl with 1 to 4 carbon atoms, or represents alkenyl with 2 to 4 carbon atoms, or represents halogenoalkyl with 1 to 4 carbon atoms and 1 to 5 identical or different halogen atoms, or represents alkoxyalkyl with in each case 1 to 4 carbon atoms in the alkoxy part and in the alkyl part, or represents dialkylaminoalkyl with up to 4 carbon atoms in the individual alkyl parts, or represents alkoxycarbonylalkyl with in each case 1 to 4 carbon atoms in the alkoxy part and in the alkyl part, or hydroxycarbonylalkyl with 1 to 4 carbon atoms in the alkyl part, or aminocarbonylalkyl, alkylaminocarbonylalkyl or dialkylaminocarbonylalkyl with in each case 1 to 4 carbon atoms in each alkyl part, or represents cyanoalkyl with 1 to 4 carbon atoms in the alkyl part, or phenylalkyl which has 1 to 4 carbon atoms in the alkyl part and is optionally mono-, di or trisubstituted in the phenyl part by identical or different substituents, or phenyl which is mono-, di- or trisubstituted by identical or different substituents, possible substituents in each case being the phenyl substituents already mentioned for R, or represents cycloalkyl which has 3 to 7 carbon atoms and is optionally substituted by identical or different substituents, possible substituents being halogen and alkyl with 1 to 4 carbon atoms, or represents alkylcarbonylamino or alkoxycarbonylamino with in each case 1 to 4 carbon atoms in the alkyl part and in the alkoxy part, or represents aminocarbonylamino, alkylaminocarbonylamino and dialkylaminocarbonylamino with in each case 1 to 4 carbon atoms in the individual alkyl parts, and also represents formylamino, or represents the grouping

$-N=CH-R^{IV}$ ,

wherein

$R^{IV}$ represents straight-chain or branched alkyl with 1 to 4 carbon atoms, or phenyl which is optionally mono-, di- or trisubstituted by identical or different substituents, possible substituents being the phenyl substituents already mentioned for R,

or

$R^{II}$ and $R^{III}$, together with the nitrogen atom to which they are bonded, represent a 5- or 6-membered heterocyclic ring, which can optionally contain oxygen or nitrogen as further hetero atoms and can optionally be substituted by cyano, halogen, alkyl with 1 to 4 carbon atoms, hydroxycarbonyl, alkoxycarbonyl with 1 to 4 carbon atoms in the alkoxy part, aminocarbonyl, and alkylaminocarbonyl and dialkylaminocarbonyl with in each case 1 to 4 carbon atoms in the individual alkyl parts, and physiologically acceptable optionally substituted ammonium, alkali metal and alkaline earth metal salts and metal salt complexes thereof,

with the exception of compounds
in which $R^{III}$ represents alkyl,
when $R^1$ and $R^{II}$ represent hydrogen.

**2.** E-Isomers of $N^{\alpha}$-(2-cyano-2-alkoximino-acetyl)-amino acid derivatives and peptides of the formula (I) according to Claim 1, in which

R represents straight-chain or branched alkyl with 1 to 4 carbon atoms, or allyl, propargyl, cyanomethyl or cyanoethyl, or represents phenylalkyl which has 1 or 2 carbon atoms in the alkyl part and is optionally mono- or disubstituted in the phenyl part by identical or different substituents, substituents which may be mentioned being: fluorine, chlorine, cyano, nitro, hydroxyl, methyl, methoxy, methylthio, methylsulphinyl, methylsulphonyl and phenyl which is optionally mono- or disubstituted by identical or different substituents from the group comprising fluorine and chlorine; or furthermore represents cyclopropyl, cyclobutyl, cyclopentyl, cyclohexyl or cycloheptyl, in each case optionally mono- or disubstituted by identical or different substituents from the group comprising fluorine, chlorine and methyl;

$R^1$ represents hydrogen, methyl or ethyl, or represents phenyl or benzyl, in each case optionally mono- or disubstituted by identical or different substituents, possible substituents being, in particular, the phenyl substituents already mentioned for R; or furthermore represents methoxycarbonylamino or ethoxycarbonylamino;

$R^2$ represents hydrogen, methyl or ethyl;

$R^3$ represents hydrogen, straight-chain or branched alkyl with 1 to 4 carbon atoms, 1-hydroxymethyl or hydroxyethyl, or represents methoxycarbonylmethyl, methoxycarbonylethyl, ethoxycarbonylethyl, hydroxycarbonylmethyl, hydroxycarbonylethyl, aminocarbonylmethyl, aminocarbonylethyl, or represents cyanomethyl or cyanoethyl, or represents allyl, or represents propargyl, or represents cyclopropyl, cyclobutyl, cyclopentyl, cyclohexyl or cycloheptyl, in each case optionally mono- or disubstituted by identical or different substituents from the group comprising fluorine, chlorine and methyl; or furthermore represents phenylalkyl which has 1 or 2 carbon atoms in the alkyl part and is optionally mono- or disubstitued in the phenyl part by identical or different substituents, or phenyl which is mono- or disubstituted by identical or different substituents, possible substituents being the phenyl substituents already mentioned for R, or furthermore represents the grouping

$R^4-SO_n-Z-$ ,

wherein

$R^4$ represents hydrogen, methyl or ethyl, or represents phenylalkyl which has 1 or 2 carbon atoms in the alkyl part and is optionally mono-or disubstituted in the phenyl

part by identical or different substituents, possible substituents being, in particular, the phenyl substituents already mentioned for R,

n represents the number 0, 1 or 2 and

Z represents a straight-chain or branched alkylene chain with 1 or 2 carbon atoms;

$R^2$ and $R^3$, together with the carbon atom to which they are bonded, represent cyclopropylidene, and

X represents the groupings $-OR^I$ or $-NR^{II}R^{III}$, wherein

$R^I$ represents hydrogen;

$R^{II}$ represents hydrogen, methyl or ethyl;

$R^{III}$ represents methyl, ethyl, n-propyl, i-propyl, n-butyl, i-butyl, sec-butyl, tert-butyl or allyl, or represents halogenoalkyl with 1 or 2 carbon atoms and 1 to 3 identical or different halogen atoms, such as fluorine and chlorine atoms, or represents alkoxyalkyl with in each case 1 or 2 carbon atoms in the alkoxy part and in the alkyl part, or represents dialkylaminoalkyl with 1 or 2 carbon atoms in the individual alkyl parts, or represents alkoxycarbonylalkyl, hydroxycarbonylalkyl, aminocarbonylalkyl, alkylaminocarbonylalkyl or dialkylaminocarbonylalkyl with in each case 1 or 2 carbon atoms in each alkyl part, or cyanoalkyl with 1 or 2 carbon atoms in the alkyl part, or represents phenylalkyl which has 1 or 2 carbon atoms in the alkyl part and is optionally mono- or disubstituted in the phenyl part by identical or different substitents, or represents phenyl which is mono- or disubstituted by identical or different substituents, possible substituents being the phenyl substituents already mentioned for R; or represents cyclopropyl, cyclobutyl, cyclopentyl, cyclohexyl or cycloheptyl, in each case optionally mono- or disubstituted by identical or different substituents from the group comprising fluorine, chlorine and methyl, or represents alkylcarbonylamino or alkoxycarbonylamino with in each case 1 or 2 carbon atoms in the alkyl part and in the alkoxy part, or represents aminocarbonylamino, methylaminocarbonylamino, ethylaminocarbonylamino, dimethylaminocarbonylamino, diethylaminocarbonylamino, methylethylaminocarbonylamino, and represents formylamino, or represents the grouping

$-N=CH-R^{IV}$ ,

wherein

$R^{IV}$ represents methyl or ethyl, or represents phenyl which is optionally mono- or disubstituted by identical or different substituents, possible substituents being the phenyl substituents mentioned for R; or

$R^{II}$ and $R^{III}$, together with the nitrogen atom to which they are bonded, represent a 5- or 6-membered heterocyclic ring, which can optionally contain oxygen or nitrogen as further hetero atoms and can optionally be substituted by cyano, fluorine, chlorine, bromine, iodine, methyl, ethyl, i-propyl, hydroxycarbonyl, methoxycarbonyl, ethoxycarbonyl, aminocarbonyl, methylaminocarbonyl, ethylaminocarbonyl, dimethylaminocarbonyl, diethylaminocarbonyl and methylethylaminocarbonyl, and physiologically acceptable optionally substituted ammonium, alkali metal and alkaline earth metal salts and metal salt complexes thereof,

with the exception of compounds in which $R^{III}$ represents alkyl when $R^1$ and $R^{II}$ represent hydrogen.

**3.** Process for the preparation of E-isomers of $N^\alpha$-(2-cyano-2-alkoximinoacetyl)-amino acid derivatives and peptides of the formula (I)

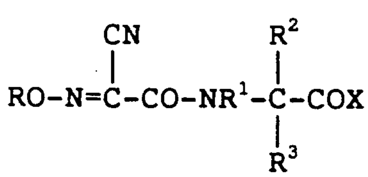

(I)

in which

R represents straight-chain or branched alkyl with 1 to 8 carbon atoms, or alkenyl or alkinyl with in each case 2 to 4 carbon atoms, or represents cyanoalkyl with 1 to 4 carbon atoms, or represents phenyl-alkyl which has 1 to 4 carbon atoms in the alkyl part and is optionally mono-, di- or trisubstituted in the phenyl part by identical or different substituents, substitutents which may be mentioned being: halogen, cyano, nitro, hydroxyl, alkyl, alkoxy, alkylthio, alkylsulphinyl and alkylsulphonyl with in each case 1 to 4 carbon atoms and phenyl which is optionally mono-, di- or trisubstituted by identical or different halogen substituents, or represents cycloalkyl which has 3 to 7 carbon atoms and is optionally mono-, di- or trisubstituted by identical or different substituents, substituents which may be mentioned being halogen and alkyl with 1 to 4 carbon atoms;

$R^1$ represents hydrogen, or straight-chain or branched alkyl with 1 to 4 carbon atoms, or represents phenyl or benzyl, in each case optionally mono-, di- or trisubstituted by identical or different substituents, possible substituents being the phenyl substituents already mentioned for R, or represents alkoxycarbonylamino with 1 to 4 carbon atoms in the alkyl part,

$R^2$ represents hydrogen, or straight-chain or branched alkyl with 1 to 4 carbon atoms;

$R^3$ represents hydrogen, or straight-chain or branched alkyl with 1 to 6 carbon atoms, or represents straight-chain or branched hydroxyalkyl with 1 to 4 carbon atoms, or represents alkoxycarbonylalkyl with in each case 1 to 4 carbon atoms in the alkoxy part and in the alkyl part, or represents hydroxycarbonylalkyl and aminocarbonylalkyl with in each case 1 to 4 carbon atoms in the alkyl part, or represents cyanoalkyl with 1 to 4 carbon atoms, or represents alkenyl or alkinyl with in each case 2 to 4 carbon atoms, or represents cycloalkyl which has 3 to 7 carbon atoms and is optionally mono-, di- or trisubstituted by identical or different substituents, substituents which may be mentioned being halogen and alkyl with 1 to 4 carbon atoms, or furthermore represents phenyl or phenylalkyl, with 1 to 4 carbon atoms in the alkyl part, optionally mono-, di- or trisubstituted in the phenyl part by identical or different substituents, substituents which may be mentioned in each case being the phenyl substutuents already mentioned for R, or represents the grouping

$R^4$-$SO_n$-Z- ,

wherein

$R^4$ represents hydrogen, or straight-chain or branched alkyl with 1 to 4 carbon atoms, or represents phenylalkyl which has 1 to 4 carbon atoms in the alkyl part and is optionally mono-, di- or trisubstituted in the phenyl part by identical or different substituents, possible substituents being the phenyl substituents already mentioned for R,

n represents the number 0, 1 or 2 and

Z represents a straight-chain or branched alkylene chain with 1 to 4 carbon atoms;

$R^2$ and $R^3$, together with the carbon atom to which they are bonded, represent cycloalkylidene with 3 to 6 carbon atoms;

X represents the groupings $-OR^I$ or $-NR^{II}R^{III}$, wherein

$R^I$ represents hydrogen,

$R^{II}$ represents hydrogen, or straight-chain or branched alkyl with 1 to 4 carbon atoms;

$R^{III}$ represents straight-chain or branched alkyl with 1 to 4 carbon atoms, or represents alkenyl with 2 to 4 carbon atoms, or represents halogenoalkyl with 1 to 4 carbon atoms and 1 to 5 identical or different halogen atoms, or represents alkoxyalkyl with in each case 1 to 4 carbon atoms in the alkoxy part and in the alkyl part, or represents dialkylaminoalkyl with up to 4 carbon atoms in the individual alkyl parts, or represents alkoxycarbonylalkyl with in each case 1 to 4 carbon atoms in the alkoxy part and in the alkyl part, or hydroxycarbonylalkyl with 1 to 4 carbon atoms in the alkyl part, or aminocarbonylalkyl, alkylaminocarbonylalkyl or dialkylaminocarbonylalkyl with in each case 1 to 4 carbon atoms in each alkyl part, or represents cyanoalkyl with 1 to 4 carbon atoms in the alkyl part, or phenylalkyl which has 1 to 4 carbon atoms in the alkyl part and is optionally mono-, di or trisubstituted in the phenyl part

by identical or different substituents, or phenyl which is mono-, di- or trisubstituted by identical or different substituents, possible substituents in each case being the phenyl substituents already mentioned for R, or represents cycloalkyl which has 3 to 7 carbon atoms and is optionally substituted by identical or different substituents, possible substituents being balogen and alkyl with 1 to 4 carbon atoms, or represents alkylcarbonylamino or alkoxycarbonylamino with in each case 1 to 4 carbon atoms in the alkyl part and in the alkoxy part, or represents aminocarbonylamino, alkylaminocarbonylamino and dialkylaminocarbonylamino with in each case 1 to 4 carbon atoms in the individual alkyl parts, and also represents formylamino, or represents the grouping

$-N=CH-R^{IV}$ ,

wherein

$R^{IV}$ represents straight-chain or branched alkyl with 1 to 4 carbon atoms, or phenyl which is optionally mono-, di- or trisubstituted by identical or different substituents, possible substituents being the phenyl substituents already mentioned for R,

or

$R^{II}$ and $R^{III}$, together with the nitrogen atom to which they are bonded, represent a 5- or 6-membered heterocyclic ring, which can optionally contain oxygen or nitrogen as further hetero atoms and can optionally be substituted by cyano, halogen, alkyl with 1 to 4 carbon atoms, hydroxycarbonyl, alkoxycarbonyl with 1 to 4 carbon atoms in the alkoxy part, aminocarbonyl, and alkylaminocarbonyl and dialkylaminocarbonyl with in each case 1 to 4 carbon atoms in the individual alkyl parts, and physiologically acceptable optionally substituted ammonium, alkali metal and alkaline earth metal salts and metal salt complexes thereof,

characterised in that

(a) amino acid derivatives of the formula (II)

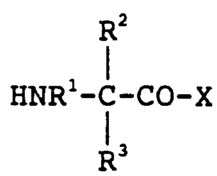

(II)

in which $R^1$, $R^2$, $R^3$ and X have the abovementioned meaning, are reacted with the carboxyl-activated derivatives of the E-isomers of the carboxylic acids of the formula (III)

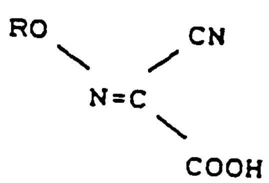

(III)

in which R has the abovementioned meaning, if appropriate in the presence of a catalyst, if appropriate in the presence of an acid-binding agent and if appropriate in the presence of a diluent, or

(b) carboxyl-activated derivatives of the E-isomers of carboxylic acids of the formula (III)

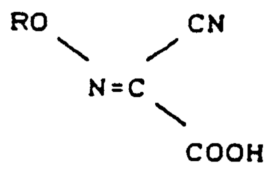

(III)

in which R has the abovementioned meaning, are reacted first with amines of the formula (IV)

$H_2NR^1$ (IV)

in which $R^1$ has the abovementioned meaning, and then with carbonyl derivatives of the formula (V)

$R^2$-CO-$R^3$ (V)

in which $R^2$ and $R^3$ have the abovementioned meanings, and finally with isonitriles of the formula (VI)

CN-$R^5$ (VI)

in which
$R^5$ represents alkyl, alkenyl, alkinyl, cyanoalkyl, alkoxycarbonylalkyl, or represents optionally substituted cycloalkyl, phenyl or phenylalkyl,
if appropriate in the presence of a diluent, or
(c) carboxyl-activated derivatives of the E-isomers of amino acids of the formula (Ia)

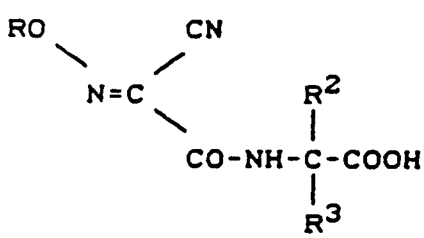

(Ia)

in which
R, $R^2$ and $R^3$ have the abovementioned meanings,
are reacted with amines of the flrmula (VII)

H-$NR^{II}R^{III}$ (VII)

in which
$R^{II}$ and $R^{III}$ have, the abovementioned meanings, if appropriate in the presence of a catalyst and if appropriate in the presence of an acid-binding agent and if appropriate in the presence of a diluent,
or

(d) E-isomers of amino acid derivatives of the formula (Ib)

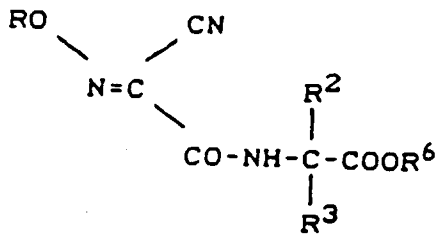

(Ib)

in which,

R, $R^2$ and $R^3$ have the abovementioned meanings and

$R^6$ represents alkyl with 1 to 4 carbon atoms, are reacted with amines of the formula (VII)

$H\text{-}NR^{II}R^{III}$ (VII)

in which

$R^{II}$ and $R^{III}$ have the abovementioned meanings,

if appropriate in the presence of a diluent, and, if appropriate, the products are then reacted to give an optionally substituted ammonium, alkali metal or alkaline earth metal salt or a metal salt complex.

**4.** Agents for combating pests, characterised in that they contain at least one E-isomer of $N^{\alpha}$-(2-cyano-2-alkoximino-acetyl)-amino acid derivative and peptide of the formula (I) according to Claims 1 to 3.

**5.** Use of E-isomers of $N^{\alpha}$-(2-cyano-2-alkoximino-acetyl)-amino acid derivatives and peptides of the formula (I) according to Claims 1 to 3 for combating pests.

**6.** Method of combating pests, characterised in that E-isomers of $N^{\alpha}$-(2-cyano-2-alkoximino-acetyl)-amino acid derivatives and peptides of the formula (I) according to Claims 1 to 3 are allowed to act on pests and/or their environment.

**7.** Process for the preparation of agents for combating pests, characterised in that E-isomers of $N^{\alpha}$-(2-cyano-2-alkoximino-acetyl)-amino acid derivatives and peptides of the formula (I) according to Claims 1 to 3 are mixed with extenders and/or surface-active agents.

**8.** Use of E-isomers of $N^{\alpha}$-(2-cyano-2-alkoximino-acetyl)-amino acid derivatives and peptides of the formula (I) according to Claim 5 for combating fungi.

**9.** E-Isomers of $N^{\alpha}$-(2-cyano-2-alkoximino-acetyl)-amino acid derivatives and peptides, characterised in that, in the formula (XI)

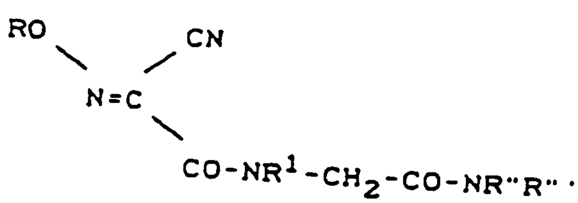

(XI)

a)

R represents methyl or benzyl,

$R^1$ represents hydrogen and

R'' is the same as R''' and represents methyl or ethyl, or

b)

R represents methyl or benzyl,

R¹ represents methyl and
R'' is the same as R''' and represents ethyl.

**10.** E-Isomers of $N^{\alpha}$-(2-cyano-2-alkoximino-acetyl)-amino acid derivatives and peptides, characterized in that, in the formula (XII)

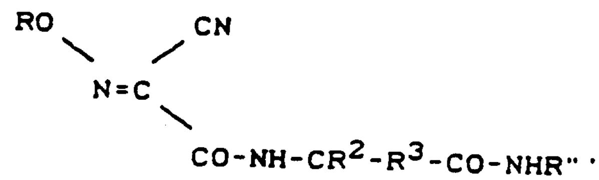

(XII)

a)
- R represents methyl, benzyl, 2-propyl, cyclohexyl, 3,4-dichlorobenzyl, ethyl, 2-propenyl or 2-propinyl,
- $R^2$, $R^3$ represent hydrogen and
- R''' represent methyl, or

b)
- R represents benzyl,
- $R^2$ represents hydrogen,
- $R^3$ represents methyl, 2-propyl or benzyl and
- R''' represents methyl, or

c)
- R represents methyl,
- $R^2$ represents methyl,
- $R^3$ represents hydrogen and
- R''' represents methyl, or

d)
- R represents methyl
- $R^2$, $R^3$ represent hydrogen and
- R''' represents benzyl, ethyl, 2-propenyl, t-butyl, 2-propyl, 2-phenylethyl, ior s-butyl.

## Revendications

**1.** E-isomères de dérivés et peptides de $N^{\alpha}$-(2-cyano-2-alkoxyiminoacétyl)-amino-acides, de formule générale (I)

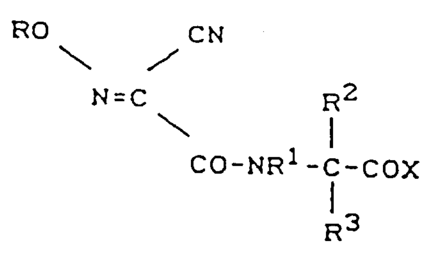

(I)

dans laquelle

R est un groupe alkyle à chaîne droite ou ramifiée ayant 1 à 8 atomes de carbone, un groupe alcényle ou alcynyle ayant chacun 2 à 4 atomes de carbone, un groupe cyanalkyle ayant 1 à 4 atomes de carbone, un groupe phénylalkyle portant éventuellement dans la partie phényle 1 à 3 substituants identiques ou différents et ayant 1 à 4 atomes de carbone dans la partie alkyle, et on mentionne alors comme substituants : un halogène, un radical cyano, nitro, hydroxy, alkyle, alkoxy, alkylsulfinyle et alkylsulfonyle, alkylthio, avec dans chaque cas 1 à 4 atomes de carbone, et un radical phényle portant

éventuellement 1 à 3 substituants halogéno identiques ou différents ; ou un groupe cycloalkyle ayant 3 à 7 atomes de carbone et portant le cas échéant 1 à 3 substituants identiques ou différents, et on mentionne alors comme substituants un halogène et un radical alkyle ayant 1 à 4 atomes de carbone ;

$R^1$ représente l'hydrogène, un groupe alkyle à chaîne droite ou ramifiée ayant 1 à 4 atomes de carbone, un groupe phényle ou un groupe benzyle portant chacun le cas échéant 1 à 3 substituants identiques ou différents, et on considère alors comme substituants les substituants du groupe phényle qui ont déjà été mentionnés pour R, ou un groupe alkoxycarbonylamino ayant 1 à 4 atomes de carbone dans la partie alkyle ;

$R^2$ représente l'hydrogène ou un groupe alkyle à chaîne droite ou ramifiée ayant 1 à 4 atomes de carbone ;

$R^3$ représente l'hydrogène ou un groupe alkyle à chaîne droite ou ramifiée ayant 1 à 6 atomes de carbone, un groupe hydroxyalkyle à chaîne droite ou ramifiée ayant 1 à 4 atomes de carbone, un groupe alkoxycarbonylalkyle ayant 1 à 4 atomes de carbone dans la partie alkoxy et dans la partie alkyle, un groupe hydroxycarbonylalkyle et un groupe aminocarbonylalkyle ayant chacun 1 à 4 atomes de carbone dans la partie alkyle, un groupe cyanalkyle ayant 1 à 4 atomes de carbone, un groupe alcényle ou un groupe alcynyle ayant chacun 2 à 4 atomes de carbone, un groupe cycloalkyle de 3 à 7 atomes de carbone portant le cas échéant 1 à 3 substituants identiques ou différents, et on mentionne alors comme substituants de préférence un halogène et un radical alkyle ayant 1 à 4 atomes de carbone, en outre un groupe phényle ou un groupe phénylalkyle ayant 1 à 4 atomes de carbone dans la partie alkyle, portant éventuellement dans la partie phényle 1 à 3 substituants identiques ou différents, et on considère alors comme substituants dans chaque cas de préférence les substituants du groupe phényle qui ont été déjà mentionnés pour R, ou le groupement

$R^4$-$SO_n$-Z-

où

$R^4$ représente l'hydrogène, un groupe alkyle à chaîne droite ou ramifiée ayant 1 à 4 atomes de carbone, un groupe phénylalkyle ayant 1 à 4 atomes de carbone dans la partie alkyle et portant le cas échéant dans la partie phényle 1 à 3 substituants identiques ou différents, et on considère alors comme substituants les substituants du groupe phényle qui ont déjà été mentionnés pour R,

n représente les nombres 0, 1 ou 2 et

Z est une chaîne alkylénique droite ou ramifiée ayant 1 à 4 atomes de carbone ;

$R^2$ et $R^3$ forment conjointement avec l'atome de carbone auquel ils sont liés un groupe cycloalkylidène ayant 3 à 6 atomes de carbone ;

X représente les groupements -$OR^I$ ou -$NR^{II}R^{III}$ où

$R^I$ représente l'hydrogène ;

$R^{II}$ est l'hydrogène ou un groupe alkyle à chaîne droite ou ramifiée ayant 1 à 4 atomes de carbone ;

$R^{III}$ est un groupe alkyle à chaîne droite ou ramifiée ayant 1 à 4 atomes de carbone, un groupe alcényle ayant 2 à 4 atomes de carbone, un groupe halogénalkyle ayant 1 à 4 atomes de carbone et 1 à 5 atomes d'halogènes identiques ou différents, un groupe alkoxyalkyle ayant 1 à 4 atomes de carbone dans la partie alkoxy et dans la partie alkyle, un groupe dialkylaminoalkyle ayant jusqu'à 4 atomes de carbone dans les parties alkyle individuelles, un groupe alkoxycarbonylalkyle ayant 1 à 4 atomes de carbone dans la partie alkoxy et dans la partie alkyle, un groupe hydroxycarbonylalkyle ayant 1 à 4 atomes de carbone dans la partie alkyle, un groupe aminocarbonylalkyle, alkylaminocarbonylalkyle ou dialkylaminocarbonylalkyle ayant chacun 1 à 4 atomes de carbone dans chaque partie alkyle, un groupe cyanalkyle ayant 1 à 4 atomes de carbone dans la partie alkyle, un groupe phénylalkyle ayant 1 à 4 atomes de carbone dans la partie alkyle et portant le cas échéant dans la partie phényle 1 à 3 substituants identiques ou différents ou un groupe phényle portant 1 à 3 substituants identiques ou différents, et on considère alors comme substituants les substituants du groupe phényle qui ont déjà été mentionnés pour R, un groupe cycloalkyle de 3 à 7 atomes de carbone portant éventuellement des substituants identiques ou différents, et on considère alors

comme substituants de préférence un halogène et un radical alkyle ayant 1 à 4 atomes de carbone, un groupe alkylcarbonylamino ou alkoxycarbonylamino ayant chacun 1 à 4 atomes de carbone dans la partie alkyle et respectivement dans la partie alkoxy, en outre un groupe aminocarbonylamino, un groupe alkylaminocarbonylamino et un groupe dialkylaminocarbonylamino ayant chacun 1 à 4 atomes de carbone dans les parties alkyle individuelles ainsi qu'un groupe formylamino ou le groupement

$-N=CH-R^{IV}$

dans lequel

$R^{IV}$ est un groupe alkyle à chaîne droite ou ramifiée ayant 1 à 4 atomes de carbone ou un groupe phényle portant le cas échéant 1 à 3 substituants identiques ou différents, et on considère alors comme substituants les substituants du groupe phényle déjà mentionnés pour R,

ou bien

$R^{II}$ et $R^{III}$ forment conjointement avec l'atome d'azote auquel ils sont liés un hétérocycle pentagonal ou hexagonal qui peut contenir le cas échéant de l'oxygène ou de l'azote comme autres hétéroatomes et qui peut être substitué éventuellement par un radical cyano, halogéno, alkyle ayant 1 à 4 atomes de carbone, hydroxycarbonyle, alkoxycarbonyle ayant 1 à 4 atomes de carbone dans la partie alkoxy, aminocarbonyle ainsi qu'alkylaminocarbonyle et dialkylaminocarbonyle avec chacun 1 à 4 atomes de carbone dans les parties alkyle individuelles,

ainsi que leurs sels d'ammonium éventuellement substitué, de métaux alcalins et de métaux alcalino-terreux ainsi que leurs complexes de sels métalliques, acceptables du point de vue physiologique, à l'exception de composés,

dans lesquels

$R^{III}$ est un groupe alkyle,
lorsque $R^1$ et $R^{II}$ représentent l'hydrogène.

**2.** E-isomères de dérivés et peptides de $N^{\alpha}$-(2-cyano-2-alkoxyiminoacétyl)-amino-acides, de formule (I) suivant la revendication 1, dans laquelle

R est un groupe alkyle à chaîne droite ou ramifiée ayant 1 à 4 atomes de carbone, un groupe allyle, propargyle, cyanométhyle, cyanéthyle, un groupe phénylalkyle ayant 1 ou 2 atomes de carbone dans la partie alkyle et portant le cas échéant dans la partie phényle 1 ou 2 substituants identiques ou différents, et on mentionne alors comme substituants : le fluor, le chlore, un radical cyano, nitro, hydroxy, méthyle, méthoxy, méthylthio, méthylsulfynyle, méthylsulfonyle et phényle portant le cas échéant 1 ou 2 substituants fluoro ou chloro identiques ou différents ; en outre un groupe cyclopropyle, cyclobutyle, cyclopentyle, cyclohexyle ou cycloheptyle, portant chacun le cas échéant 1 ou 2 substituants fluoro, chloro ou méthyle identiques ou différents ;

$R^1$ est l'hydrogène, un groupe méthyle, éthyle, un groupe phényle ou benzyle portant chacun le cas échéant 1 ou 2 substituants identiques ou différents, et on considère alors comme substituants, en particulier les substituants du groupe phényle déjà mentionnés pour R ; en outre un groupe méthoxycarbonylamino ou éthoxycarbonylamino ;

$R^2$ est l'hydrogène, un groupe méthyle ou éthyle ;

$R^3$ est l'hydrogène, un groupe alkyle à chaîne droite ou ramifiée de 1 à 4 atomes de carbone, un groupe 1-hydroxyméthyle, hydroxyéthyle, un groupe méthoxycarbonylméthyle, méthoxycarbonyléthyle, éthoxycarbonyléthyle, hydroxycarbonylméthyle, hydroxycarbonyléthyle, aminocarbonylméthyle, aminocarbonyléthyle, cyanométhyle, cyanéthyle, allyle, propargyle, un groupe cyclopropyle, cyclobutyle, cyclopentyle, cyclohexyle ou cycloheptyle portant chacun le cas échéant 1 ou 2 substituants fluoro, chloro ou méthyle identiques ou différents ; en outre un groupe phénylalkyle ayant 1 ou 2 atomes de carbone dans la partie alkyle portant éventuellement 1 ou 2 substituants identiques ou différents dans la partie phényle, ou bien un groupe phényle portant 1 ou 2 substituants identiques ou différents, et on considère alors comme substituants les substituants du groupe phényle déjà mentionnés pour R, en outre le groupement

$R^4-SO_n-Z-$

dans lequel

$R^4$ représente l'hydrogène, un groupe méthyle, éthyle ou un groupe phénylalkyle ayant 1 ou 2 atomes de carbone dans la partie alkyle et portant éventuellement dans la partie phényle 1 ou 2 substituants identiques ou différents, et on considère alors comme substituants en particulier les substituants du groupe phényle déjà mentionnés pour R,

n représente les nombres 0, 1 ou 2 et

Z est une chaîne alkylénique droite ou ramifiée ayant 1 ou 2 atomes de carbone,

$R^2$ et $R^3$ forment un groupe cyclopropylidène conjointement avec l'atome de carbone auquel ils sont liés et

X représente les groupements $-OR^I$ ou $-NR^{II}R^{III}$, dans lesquels

$R^I$ est l'hydrogène ;

$R^{II}$ est l'hydrogène, un groupe méthyle ou éthyle ;

$R^{III}$ est un groupe méthyle, éthyle, n-propyle, isopropyle, n-butyle, isobutyle, sec.butyle, tertiobutyle, allyle, un groupe halogénalkyle ayant 1 ou 2 atomes de carbone et 1 à 3 atomes d'halogènes identiques ou différents tels que des atomes de fluor et de chlore, un groupe alkoxyalkyle ayant 1 ou 2 atomes de carbone dans la partie alkoxy ainsi que dans la partie alkyle, un groupe dialkylaminoalkyle ayant 1 ou 2 atomes de carbone dans les parties alkyle individuelles, un groupe alkoxycarbonylalkyle, hydroxycarbonylalkyle, aminocarbonylalkyle, alkylaminocarbonylalkyle ou dialkylaminocarbonylalkyle ayant chacun 1 ou 2 atomes de carbone dans chaque partie alkyle, un groupe cyanalkyle ayant 1 ou 2 atomes de carbone dans la partie alkyle ; en outre un groupe phénylalkyle ayant 1 ou 2 atomes de carbone dans la partie alkyle et portant la cas échéant dans la partie phényle 1 ou 2 substituants identiques ou différents, un groupe phényle portant 1 ou 2 substituants identiques ou différents, et on considère alors comme substituants dans chaque cas les substituants du groupe phényle déjà mentionnés pour R ; un groupe cyclopropyle, cyclobutyle, cyclopentyle, cyclohexyle ou cycloheptyle portant chacun le cas échéant 1 ou 2 substituants fluoro, chloro ou méthyle identiques ou différents, un groupe alkylcarbonylamino ou alkoxycarbonylamino ayant chacun 1 ou 2 atomes de carbone dans la partie alkyle ou respectivement dans la partie alkoxy, un groupe aminocarbonylamino, méthylaminocarbonylamino, éthylaminocarbonylamino, diméthylaminocarbonylamino, diéthylaminocarbonylamino, méthyléthylaminocarbonylamino ainsi qu'un groupe formylamino ou le groupement

$-N=CH-R^{IV}$

dans lequel

$R^{IV}$ est un groupe méthyle, éthyle, phényle portant le cas échéant 1 ou 2 substituants identiques ou différents, et on considère alors comme substituants les substituants du groupe phényle déjà mentionnés pour R ; ou bien

$R^{II}$ et $R^{III}$ forment conjointement avec l'atome d'azote auquel ils sont liés un hétérocycle pentagonal ou hexagonal qui peut contenir le cas échéant de l'oxygène ou de l'azote comme autres hétéroatomes et qui peut être substitué le cas échéant par un radical cyano, du fluor, du chlore, du brome, de l'iode, un radical méthyle, éthyle, isopropyle, hydroxycarbonyle, méthoxycarbonyle, éthoxycarbonyle, aminocarbonyle, méthylaminocarbonyle, éthylaminocarbonyle, diméthylaminocarbonyle, diéthylaminocarbonyle ainsi que méthyléthylaminocarbonyle, ainsi que leurs sels d'ammonium éventuellement substitué, de métaux alcalins et de métaux alcalino-terreux ainsi que leurs complexes de sels métalliques, acceptables du point de vue physiologique, à l'exception de composés dans lesquels $R^{III}$ est un groupe alkyle lorsque $R^1$ et $R^{II}$ représentent l'hydrogène.

**3.** Procédé de production de E-isomères de dérivés et peptides d'acides $N^{\alpha}$-(2-cyano-2-alkoxyiminoacétyl)-aminoacides, de formule (I)

```
        CN          R2
        |           |
RO-N=C-CO-NR1-C-COX                (I)
                    |
                    R3
```

dans laquelle

R est un groupe alkyle à chaîne droite ou ramifiée ayant 1 à 8 atomes de carbone, un groupe alcényle ou alcynyle ayant chacun 2 à 4 atomes de carbone, un groupe cyanalkyle ayant 1 à 4 atomes de carbone, un groupe phénylalkyle portant éventuellement dans la partie phényle 1 à 3 substituants identiques ou différents et ayant 1 à 4 atomes de carbone dans la partie alkyle, et on mentionne alors comme substituants : un halogène, un radical cyano, nitro, hydroxy, alkyle, alkoxy, alkylsulfinyle et alkylsulfonyle, alkylthio, avec dans chaque cas 1 à 4 atomes de carbone, et un radical phényle portant éventuellement 1 à 3 substituants halogéno identiques ou différents ; ou un groupe cycloalkyle ayant 3 à 7 atomes de carbone et portant le cas échéant 1 à 3 substituants identiques ou différents, et on mentionne alors comme substituants de préférence un halogène et un radical alkyle ayant 1 à 4 atomes de carbone ;

$R^1$ représente l'hydrogène, un groupe alkyle à chaîne droite ou ramifiée ayant 1 à 4 atomes de carbone, un groupe phényle ou un groupe benzyle portant chacun le cas échéant 1 à 3 substituants identiques ou différents, et on considère alors comme substituants de préférence les substituants du groupe phényle qui ont déjà été mentionnés pour R, ou un groupe alkoxycarbonylamino ayant 1 à 4 atomes de carbone dans la partie alkyle ;

$R^2$ représente l'hydrogène ou un groupe alkyle à chaîne droite ou ramifiée ayant 1 à 4 atomes de carbone ;

$R^3$ représente l'hydrogène ou un groupe alkyle à chaîne droite ou ramifiée ayant 1 à 6 atomes de carbone, un groupe hydroxyalkyle à chaîne droite ou ramifiée ayant 1 à 4 atomes de carbone, un groupe alkoxycarbonylalkyle ayant 1 à 4 atomes de carbone dans la partie alkoxy et dans la partie alkyle, un groupe hydroxycarbonylalkyle et un groupe aminocarbonylalkyle ayant chacun 1 à 4 atomes de carbone dans la partie alkyle, un groupe cyanalkyle ayant 1 à 4 atomes de carbone, un groupe alcényle ou un groupe alcynyle ayant chacun 2 à 4 atomes de carbone, un groupe cycloalkyle de 3 à 7 atomes de carbone portant le cas échéant 1 à 3 substituants identiques ou différents, et on mentionne alors comme substituants de préférence un halogène et un radical alkyle ayant 1 à 4 atomes de carbone, en outre un groupe phényle ou un groupe phénylalkyle ayant 1 à 4 atomes de carbone dans la partie alkyle, portant éventuellement dans la partie phényle 1 à 3 substituants identiques ou différents, et on considère alors comme substituants dans chaque cas les substituants du groupe phényle qui ont été déjà mentionnés pour R, ou le groupement

$R^4-SO_n-Z-$

où

$R^4$ représente l'hydrogène, un groupe alkyle à chaîne droite ou ramifiée ayant 1 à 4 atomes de carbone, un groupe phénylalkyle ayant 1 à 4 atomes de carbone dans la partie alkyle et portant le cas échéant dans la partie phényle 1 à 3 substituants identiques ou différents, et on considère alors comme substituants les substituants du groupe phényle qui ont déjà été mentionnés pour R,

n représente les nombres 0, 1 ou 2 et

Z est une chaîne alkylénique droite ou ramifiée ayant 1 à 4 atomes de carbone ;

$R^2$ et $R^3$ forment conjointement avec l'atome de carbone auquel ils sont liés un groupe cycloalkylidène ayant 3 à 6 atomes de carbone ;

X représente les groupements $-OR^I$ ou $-NR^{II}R^{III}$ où

$R^I$ représente l'hydrogène ;

$R^{II}$ est l'hydrogène ou un groupe alkyle à chaîne droite ou ramifiée ayant 1 à 4 atomes de carbone ;

$R^{III}$ est un groupe alkyle à chaîne droite ou ramifiée ayant 1 à 4 atomes de carbone, un groupe alcényle ayant 2 à 4 atomes de carbone, un groupe halogénalkyle ayant 1 à 4 atomes de carbone et 1 à 5 atomes d'halogène identiques ou différents, un groupe alkoxyalkyle ayant 1 à 4 atomes de carbone dans la partie alkoxy et dans la partie alkyle, un groupe dialkylaminoalkyle ayant jusqu'à 4 atomes de carbone dans les parties alkyle individuelles, un groupe alkoxycarbonylalkyle ayant 1 à 4 atomes de carbone dans la partie alkoxy et dans la partie alkyle, un groupe hydroxycarbonylalkyle ayant 1 à 4 atomes de carbone dans la partie alkyle, un groupe aminocarbonylalkyle, alkylaminocarbonylalkyle ou dialkylaminocarbonylalkyle ayant chacun 1 à 4 atomes de carbone dans chaque partie alkyle, un groupe cyanalkyle ayant 1 à 4 atomes de carbone dans la partie alkyle, un groupe phénylalkyle ayant 1 à 4 atomes de carbone dans la partie alkyle et portant le cas échéant dans la partie phényle 1 à 3 substituants identiques ou différents ou un groupe phényle portant 1 à 3 substituants identiques ou différents, et on considère alors comme substituants les substituants du groupe phényle qui ont déjà été mentionnés pour R, un groupe cycloalkyle de 3 à 7 atomes de carbone portant éventuellement des substituants identiques ou différents, et on considère alors comme substituants un halogène et un radical alkyle ayant 1 à 4 atomes de carbone, un groupe alkylcarbonylamino ou alkoxycarbonylamino ayant chacun 1 à 4 atomes de carbone dans la partie alkyle et respectivement dans la partie alkoxy, en outre un groupe aminocarbonylamino, un groupe alkylaminocarbonylamino et un groupe dialkylaminocarbonylamino ayant chacun 1 à 4 atomes de carbone dans les parties alkyle individuelles ainsi qu'un groupe formylamino ou le groupement

$$-N=CH-R^{IV}$$

dans lequel

$R^{IV}$ est un groupe alkyle à chaîne droite ou ramifiée ayant 1 à 4 atomes de carbone ou un groupe phényle portant le cas échéant 1 à 3 substituants identiques ou différents, et on considère alors comme substituants les substituants du groupe phényle déjà mentionnés pour R,

ou bien

$R^{II}$ et $R^{III}$ forment conjointement avec l'atome d'azote auquel ils sont liés un hétérocycle pentagonal ou hexagonal qui peut contenir le cas échéant de l'oxygène ou de l'azote comme autres hétéroatomes et qui peut être substitué éventuellement par un radical cyano, halogéno, alkyle ayant 1 à 4 atomes de carbone, hydroxycarbonyle, alkoxycarbonyle ayant 1 à 4 atomes de carbone dans la partie alkoxy, aminocarbonyle ainsi qu'alkylaminocarbonyle et dialkylaminocarbonyle avec chacun 1 à 4 atomes de carbone dans les parties alkyle individuelles,

ainsi que leurs sels d'ammonium éventuellement substitué, de métaux alcalins et de métaux alcalino-terreux ainsi que leurs complexes de sels métalliques, acceptables du point de vue physiologique, à l'exception de composés, dans lesquels

$R^{III}$ est un groupe alkyle,
lorsque $R^1$ et $R^{II}$ représentent l'hydrogène,

caractérisé en ce que

(a) on fait réagir des dérivés d'amino-acides de formule (II)

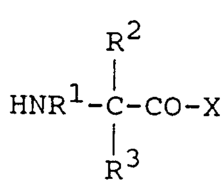

(II)

dans laquelle $R^1$, $R^2$, $R^3$ et X ont la définition indiquée ci-dessus, avec les dérivés carboxy-activés des E-isomères d'acides carboxyliques de formule (III)

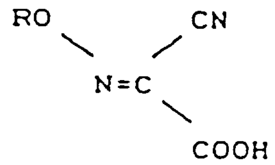

(III)

dans laquelle R a la définition indiquée ci-dessus, le cas échéant en présence d'un catalyseur, en la présence éventuelle d'un accepteur d'acide et le cas échéant en présence d'un diluant,
ou bien
(b) on fait réagir des dérivés carboxy-activés des E-isomères d'acides carboxyliques de formule (III)

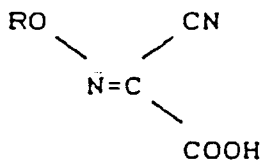

(III)

dans laquelle R a la définition indiquée ci-dessus,
tout d'abord avec des amines de formule (IV)

$H_2NR^1$ (IV)

dans laquelle $R^1$ a la définition indiquée ci-dessus,
puis avec des dérivés carbonyliques de formule (V)

$R^2$-CO-$R^3$ (V)

dans laquelle $R^2$ et $R^3$ ont les définitions indiquées ci-dessus,
et finalement avec des isonitriles de formule (VI)

CN-$R^5$ (VI)

dans laquelle

$R^5$ représente un groupe alkyle, alcényle, alcynyle, cyanalkyle, alkoxycarbonylalkyle ou un groupe cycloalkyle, phényle ou phénylalkyle éventuellement substitué,
en la présence éventuelle d'un diluant, ou bien

(c) on fait réagir des dérivés carboxy-activés des E-isomères d'amino-acides de formule (Ia)

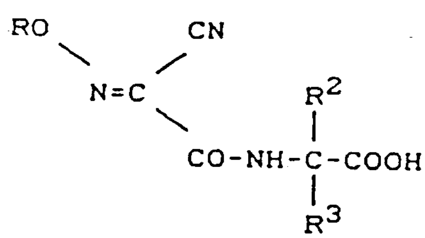

(Ia)

dans laquelle

R, $R^2$ et $R^3$ ont les définitions indiquées ci-dessus,
avec des amines de formule (VII)

H-$NR^{II}R^{III}$ (VII)

dans laquelle

$R^{II}$ et $R^{III}$ ont les définitions indiquées ci-dessus, le cas échéant en présence d'un catalyseur et en la présence éventuelle d'un accepteur d'acide et le cas échéant en présence d'un diluant,

ou bien

(d) on fait réagir des E-isomères de dérivés d'aminoacides de formule (Ib)

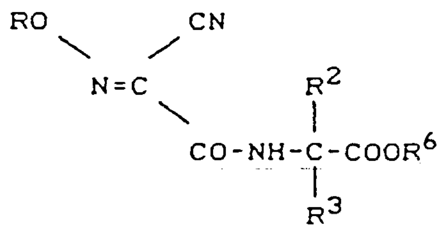

(Ib)

dans laquelle

$R$, $R^2$ et $R^3$ ont les définitions indiquées ci-dessus et

$R^6$ est un groupe alkyle ayant 1 à 4 atomes de carbone, avec des amines de formule (VII)

$H\text{-}NR^{II}R^{III}$ (VII)

dans laquelle

$R^{II}$ et $R^{III}$ ont les définitions indiquées ci-dessus,

le cas échéant en présence d'un diluant et on transforme ensuite éventuellement le produit en un sel d'ammonium éventuellement substitué, de métal alcalin ou de métal alcalino-terreux ou en un complexe de sel métallique.

**4.** Compositions pesticides, caractérisées par une teneur en au moins un E-isomère de dérivés et peptides de $N^{\alpha}$-(2-cyano-2-alkoxyiminoacétyl)-amino-acides de formule (I) suivant les revendications 1 à 3.

**5.** Utilisation de E-isomères de dérivés et peptides de $N^{\alpha}$-(2-cyano-2-alkoxyiminoacétyl)-amino-acides de formule (I) suivant les revendications 1 à 3 pour combattre des parasites.

**6.** Procédé pour combattre des parasites, caractérisé en ce qu'on fait agir des E-isomères de dérivés et peptides de $N^{\alpha}$-(2-cyano-2-alkoxyiminoacétyl)-amino-acides de formule (I) suivant les revendications 1 à 3 sur des parasites et/ou sur leur milieu.

**7.** Procédé de préparation de compositions pesticides, caractérisé en ce qu'on mélange des E-isomères de dérivés et peptides de $N^{\alpha}$-(2-cyano-2-alkoxyiminoacétyl)-amino-acides de formule (I) suivant les revendications 1 à 3 avec des diluants et/ou des agents tensio-actifs.

**8.** Utilisation de E-isomères de dérivés et peptides de $N^{\alpha}$-(2-cyano-2-alkoxyiminoacétyl)-amino-acides de formule (I) suivant la revendication 5 pour combattre des champignons.

**9.** E-isomères de dérivés et peptides d'acides $N^{\alpha}$-(2-cyano-2-alkoxyiminoacétyl)-amino-acides, caractérisés en ce que dans la formule (XI)

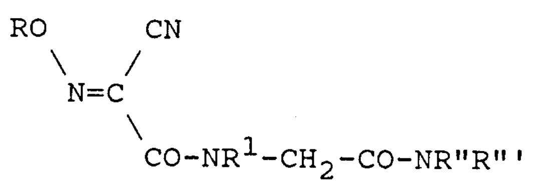

(XI)

a)

R est un groupe méthyle ou benzyle,

R[1] est l'hydrogène et
R'' identique à R''' est un groupe méthyle ou éthyle, ou bien

b)
R est un groupe méthyle ou benzyle,
$R^1$ est un groupe méthyle et
R'' identique à R''' est un groupe éthyle.

**10.** E-isomères de dérivés et peptides de $N^{\alpha}$-(2-cyano-2-alkoxyiminoacétyl)-amino-acides, caractérisés en ce que dans la formule (XII)

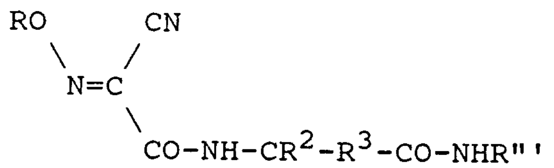

(XII)

a)
R est un groupe méthyle ou benzyle, 2-propyle, cyclohexyle, 3,4-dichlorobenzyle, éthyle, 2-propényle ou 2-propynyle,
$R^2$, $R^3$ représentent de l'hydrogène et
R''' est un groupe méthyle, ou bien

b)
R est un groupe benzyle,
$R^2$ est l'hydrogène
$R^3$ est un groupe méthyle, 2-propyle ou benzyle et
R''' est un groupe méthyle, ou bien

c)
R est un groupe méthyle,
$R^2$ est un groupe méthyle
$R^3$ est l'hydrogène et
R''' est un groupe méthyle, ou bien

d)
R est un groupe méthyle,
$R^2$, $R^3$ sont de l'hydrogène et
R''' est un groupe benzyle, éthyle, 2-propényle, tertiobutyle, 2-propyle, 2-phényléthyle, isobutyle ou sec.-butyle.